# EUROPEAN PATENT SPECIFICATION

(11) **EP 3 125 759 B1**
(45) Date of publication and mention of the grant of the patent: **06.01.2021**
(21) Application number: 15769971.1
(22) Date of filing: 27.03.2015
(51) Int. Cl.: A61B 5/107, A61B 6/02, A61B 90/00, A61B 34/20, A61B 5/06, A61B 6/03, A61B 17/16, A61B 50/26, A61B 34/10

(54) **COMPUTER AIDED SURGICAL NAVIGATION AND PLANNING IN IMPLANTOLOGY**
RECHNERGESTÜTZTE CHIRURGISCHE NAVIGATION UND PLANUNG IN DER IMPLANTOLOGIE
PLANIFICATION ET NAVIGATION CHIRURGICALES ASSISTÉES PAR ORDINATEUR EN IMPLANTOLOGIE

(30) Priority: 27.03.2014 AU 2014901095
(43) Date of publication of application: 08.02.2017
(73) Proprietor: Bresmedical Pty Limited, Sydney, New South Wales 2565 (AU); Alma Mater Studiorum - Università di Bologna, 40126 Bologna (IT)
(72) Inventor: TARASCHI, Valerio, Sydney, New South Wales 2565 (AU); PELLEGRINO, Gerardo, 40126 Bologna (IT)
(74) Representative: Zinkler, Franz
(86) International application number: PCT/AU2015/050134
(87) International publication number: WO 2015/143508

(56) References cited:
- EP-B1- 0 922 438
- EP-B1- 1 103 229
- WO-A1-2005/000139
- WO-A1-2013/080131
- WO-A2-2010/096419
- WO-A2-2010/144402
- US-B1- 6 298 262

## Description

### Technical Field

The present invention generally relates to computer-implemented or computer-aided methods and/or systems for use in medical procedures and surgery. More particularly, embodiments of the present invention relate to computer-implemented methods, systems and/or devices for surgery, planning of surgery and/or navigation during surgery.

### Background

Image-Guided Surgery (IGS) is a technique appropriate to be used in a variety of medical procedures, for example by orthopedic, oral-maxillofacial, brain, sinus and spinal surgeons to help clarify complex anatomy encountered during surgical interventions or procedures. However, although use of IGS is a state-of-the-art approach, IGS is not of itself the standard of care. IGS is presently used at the discretion of the operating surgeon and is not investigational. Other example fields of application for IGS include revision surgery, extended frontal sinus surgery, tumor resection, skull base surgery and endoneurosurgery. For example, in sinus surgery, IGS is used to identify complex anatomy in procedures such as revision sinus surgery, distorted sinus surgery, extensive sino-nasal polyposis, surgery relating to disease that abuts the skull base, optic nerve or carotid artery, and more.

In oral implantology, by way of example, poor implant positioning increases the risk of implant failure due to mechanical overload of the surrounding bone tissues. The efficacy of IGS, or Computer-Aided Surgery (CAS), has been proved and is generally accepted. Different types of IGS or CAS systems can be used for pre-operative planning, surgical navigation and to assist in performing medical procedures.

In a specific application, again by way of example only, the ability to generate three-dimensional volumetric representations or images of the maxillofacial area has allowed surgeons to evaluate a patient's anatomy before surgery and assist in planning for the placement of implants in ideal or preferred positions. However, the ability to transfer image information to surgical reality has been, and continues to be, one of the most challenging problems of IGS or CAS, for example in implant dentistry.

Despite some IGS or CAS approaches being available, there is a continuing need to improve computer-implemented methods, systems and/or devices for image based or assisted surgical planning and/or navigation. For example, in current approaches which are for mechanical guidance via split systems or visualization of a surgeon's instrument via tracking, there is currently a lack of any suitable or sufficient control or display by the tracking system on possible misplacement or positioning of, for example, a drilling template.

The reference in this specification to any prior publication (or information derived from the prior publication), or to any matter which is known, is not, and should not be taken as an acknowledgment or admission or any form of suggestion that the prior publication (or information derived from the prior publication) or known matter forms part of the common general knowledge in the field of endeavour to which this specification relates.

WO 2010/096419 A2 discloses a system for image guided surgery. The system provides information to physicians about procedures they are performing, the devices (such as ablation needles, ultrasound wands or probes, scalpels, cauterizers, etc.) they are using during the procedure, the relative emplacements or poses of these devices, prediction information for those devices, and other information.

WO 2005/000139 A1 teaches a system for navigation within a surgical field, wherein a micro-camera is provided in a hand-held navigation probe tracked by a tracking system. This enables navigation within an operative scene by viewing real-time images from the viewpoint of the micro-camera within the navigation probe, which are overlaid with computer generated 3D graphics depicting structures of interest generated from pre-operative scans. Various transparency settings of the camera image and the superimposed 3D graphics can enhance the depth perception, and distances between a tip of the probe and any of the superimposed 3D structures along a virtual ray extending from the probe tip can be dynamically displayed in the combined image. A virtual interface can be displayed adjacent to the combined image on a system display, thus facilitating interaction with various navigation related functions.

WO 2013/080131 A1 discloses a planning system including a processor and memory coupled to the processor which stores a planning module. A user interface is coupled to the processor and configured to permit a user to select a path through a pathway system. The planning module is configured to upload one or more slices of an image volume corresponding to a user-controlled cursor point guided using the user interface such that as the path is navigated the one or more slices are updated in accordance with a depth of the cursor point in the path.

### Summary

This Summary is provided to introduce a selection of concepts in a simplified form that are further described below in the Preferred Embodiments. This Summary is not intended to identify key features or essential features of the claimed subject matter, nor is it intended to be used to limit the scope of the claimed subject matter.

The invention provides a system for surgical navigation in oral surgery according to claim 1 and a computer-implemented method for planning of oral surgery according to claim 8.

Further developments of the invention are defined in the dependent claims.

Aspects, features or advantages of the present invention allow preoperative planning and/or intraoperative template guidance or computer-aided navigation for surgery. Various embodiments of the present invention relate to computer-implemented or computer-aided methods, systems and/or devices or apparatus for surgery, planning of surgery and/or navigation during surgery. Example embodiments include a computer-implemented method of surgery, surgical planning and/or surgical navigation. An example embodiment also includes a software application or part thereof. Other example embodiments include devices or apparatus for use in surgery, planning of surgery and/or navigation during surgery. Also provided is an image guided or based surgical navigation and/or surgical planning system. Embodiments also relate to combinations of the methods, systems and/or devices.

In one aspect there is provided a computer-implemented method or system for a surgical navigation procedure or surgical planning, which provides one or more, preferably multiple, two-dimensional volume slices represented as one or more slice views to a user, where the one or more slice views are updated, preferably automatically, by movement of the surgical instrument or by the virtually planned position of a surgical insert.

In another aspect there is provided a system for surgical navigation or planning, comprising: at least one processing system for: obtaining a three-dimensional (3D) anatomical representation of part of a patient; and producing at least one two-dimensional volume slice view of the three-dimensional anatomical representation, the at least one two-dimensional volume slice view based on a physical position or axial orientation of a surgical instrument or a virtual position or axial orientation of a surgical implant; and a display device for displaying the at least one two-dimensional volume slice view to a user; wherein, the at least one two-dimensional volume slice view to be displayed is updated by movement of the physical position or axial orientation of the surgical instrument during surgical navigation, or by movement of the virtual position or axial orientation of the surgical implant during surgical planning.

In another aspect there is provided a computer-implemented method for surgical navigation or planning, the method comprising: obtaining, by at least one computer system, a three-dimensional (3D) anatomical representation of part of a patient; producing, by the at least one computer system, at least one two-dimensional volume slice view of the three-dimensional anatomical representation, the at least one two-dimensional volume slice view based on a physical position or axial orientation of a surgical instrument, or a virtual position or axial orientation of a surgical implant; displaying, by a display device, the at least one two-dimensional volume slice view to a user; and, updating, by the at least one computer system, the displayed at least one two-dimensional volume slice view based on movement of the physical position or axial orientation of the surgical instrument during surgical navigation, or by movement of the virtual position or axial orientation of the surgical implant during surgical planning.

In various example but non-limiting aspects, there is provided:
1. A computer-implemented method of surgical planning.
2. A computer-implemented method of image guided surgical navigation.
3. A surgical navigation system using image guided procedures.
4. A surgical navigation system including one or more hardware components which allow real-time imaging of a surgical instrument relative to a patient's anatomy.
5. A computer-implemented method for precision placement of implants during surgery by real-time imaging of a surgical instrument relative to a patient's anatomy.
6. A computer-implemented method providing an integrated process for bone drilling and implant insertion in a patient with the use of an automatically generated surgical template.
7. A computer-implemented method providing a compound view of a virtual surgical guide and a patient's anatomical profile.
8. A drill bit and an extender for use in a surgical navigation system, where the drill bit and the extender constrain the drilling axis and drilling depth.
9. A drill bit and a collar for use in a surgical navigation system, where the drill bit and the collar constrain the drilling axis and drilling depth.
10. A connector for use in a surgical navigation system, where the connector is able to receive radiopaque material and support a reference markers tool, and the connector is able to be attached to an implant, which enables a determination of the physical position of the radiopaque material with respect to the patient.
11. A computer-implemented method for surgical navigation where the user is provided with a compound view on a display device of the position and axial orientation of a surgical instrument relative to an anatomical image of a patient.
12. A computer-implemented method for a surgical navigation procedure which provides two dimensional volume slices represented as views to a user, which are automatically updated by movement of the surgical instrument.

In other example embodiments, a surgical navigation system includes one or more software or computer-implemented components which allow virtual implant planning, generate patient's specific surgical templates and receive real-time data from a tracking device or apparatus, for example a surgical tracker. The surgical navigation system can also include one or more hardware components which allow real-time imaging of one or more surgical instruments onto or over one or more images of a patient's anatomy.

In another aspect there is provided a system which puts into effect a computer-implemented method for improving the precision and safety of image based or assisted surgery, for example precision placement of bone implants during surgery.

In another aspect the system and/or computer-implemented method allows two approaches to be integrated or otherwise provided together, such as in combination, for example a bone drilling procedure and an implant insertion procedure. In a non-limiting example, a first approach could use stereo lithographic splints manufactured according to a pre-operation implant planning stage, and a second approach could use real-time or live data from a stereoscopic scanner connected to a processing system (e.g. a computer) to guide a surgeon to accurately transfer the implant, using a displayed pre-planned implant position, into a bone implant socket(s).

### Brief Description of Figures

Example embodiments are apparent from the following description, which is given by way of example only, of at least one preferred but non-limiting embodiment, described in connection with the accompanying figures.
Fig. 1 is a functional block diagram of an example surgical navigation system.
Fig. 2 is a functional block diagram of an example processing system that can be utilised to embody or give effect to a particular embodiment.
Figs. 3a and 3b are a flow diagram of an example computer-implemented method/process which allows Computer Assisted Surgery (CAS) to be performed using drill guide generation or live tracking navigation.
Fig. 4 is a flow diagram of an example computer-implemented method/process providing a navigation mode.
Fig. 5 is a flow diagram of an example computer-implemented method/process for a surgical procedure using the example surgical navigation system.
Fig. 6 is an isometric view of an example main connector for fiducial and reference markers holders for partially edentulous patients.
Fig. 7 shows two isometric views of an example main connector for fiducial and reference markers holders for completely edentulous patients.
Fig. 8 is an isometric view of an example reference markers holder (or tool), which includes spherical reference markers and sockets for calibration positioned along the main axis of the holder
Fig. 9 is an isometric view of another example reference markers holder (or tool), which includes spherical reference markers and sockets for calibration positioned about the holder.
Fig. 10 is an isometric view of another example reference markers holder (or tool), which includes spherical reference markers.
Fig. 11 is an isometric view of another example reference markers holder (or tool), which includes spherical reference markers.
Fig. 12 is an isometric view of an example calibration device for surgical instruments, also including an example long calibration prober and an example short calibration prober in a functional position.
Fig. 13 illustrates an example reference markers holder (or tool) mounted on an example handle of a surgical instrument.
Fig. 14 illustrates an example reference markers holder (or tool) mounted on an example surgical instrument, which is being calibrated using an example reference markers holder by way of touching one or more sockets for calibration positioned about the holder.
Fig. 15 illustrates an example reference markers holder (or tool) mounted on an example piezo-electric surgical instrument.
Fig. 16 illustrates an example sterilisable calibration tool suitable for piezo-electric surgical instruments.
Fig. 17 illustrates another example sterilisable calibration tool suitable for piezo-electric surgical instruments.
Fig. 18 illustrates an example disposable calibration tool.
Fig. 19 shows (a) top and (b) bottom isometric views of another example fiducial markers plate with conical socket guides for fiducial markers and holes for simple insertion of radiopaque material.
Fig. 20 shows (a) bottom and (b) top isometric views of an example fiducial markers plate with socket guides for fiducial markers and holes for insertion of radiopaque material.
Fig. 21 illustrates an example assembly for fiducial markers and reference markers in a case of an edentulous patient, including a main connector, fiducial markers holder and reference markers holder.
Fig. 22 illustrates an example assembly for fiducial markers and reference markers in a case of a patient's reference tool connected to a fiducial tray.
Figs. 23 to 25 illustrate (a) top and (b) bottom isometric views for different example fiducial trays for partially edentulous patients.
Fig. 26 illustrates example drilling and implant devices providing an example drill set, such as extended drill shafts, drill bits and implants, and also shows example pre-drilling and drilling stages, as well as an example implant stage. The stages are part of an example guided drilling procedure.
Fig. 27 illustrates example extenders and the use of the extenders with the example drill set from Fig. 26.
Figs. 28 and 29 are sample screenshots from an example software application showing a live-tracked position of the surgical drill tip within the patient's anatomy.
Fig. 30 is a sample screenshot from the example software application showing a virtual planning phase of surgery.
Fig. 31 is a sample screenshot from the example software application in which the interface for the main virtual implant planning stage is displayed.
Figs. 32 and 33 are sample screenshots from the example software application taken during a main navigation operation.
Fig. 34 illustrates an example system or integrated device for surgical planning and/or navigation.

### Preferred Embodiments

The following modes, given by way of example only, are described in order to provide a more precise understanding of the subject matter of a preferred embodiment or embodiments. In the figures, incorporated to illustrate features of an example embodiment, like reference numerals are used to identify like parts throughout the figures.

An example embodiment provides a computer-implemented method of surgical planning and/or navigation, for example as, or at least partially as, a software application (i.e. a computer program). A surgical navigation system is also provided that includes software or computer-implemented components which allow virtual implant planning, and receive real-time data from a surgical tracker (i.e. a reference markers tracking device). The surgical navigation system can also include one or more hardware components which allow real-time imaging of one or more surgical instruments onto or over two-dimensional (2D) or three-dimensional (3D) representations or images of a patient's anatomy. The system and computer-implemented method improve the precision and safety of image based surgery, for example precision placement of bone implants during surgery. A software application is used to manage drill-guide assisted surgery and image-guided surgery. This provides a method and system for Computer Assisted Surgery (CAS) which improves accuracy and safety of a variety of surgical procedures. Various devices for use in surgery, surgical planning or surgical navigation are also provided.

The computer-implemented method, in the example form of a software application but which could be implemented as hardware or firmware, can be used to generate a virtual implant insertion on a patient's scanned (i.e. imaged) anatomical structures. The topology of the implant or implants allow the software application to automatically generate the design of a drill guide for implant socketing. Image guided surgery also requires hardware components which can be provided as part of an integrated solution including the software application, or aspects or parts thereof.

### Surgical Navigation System

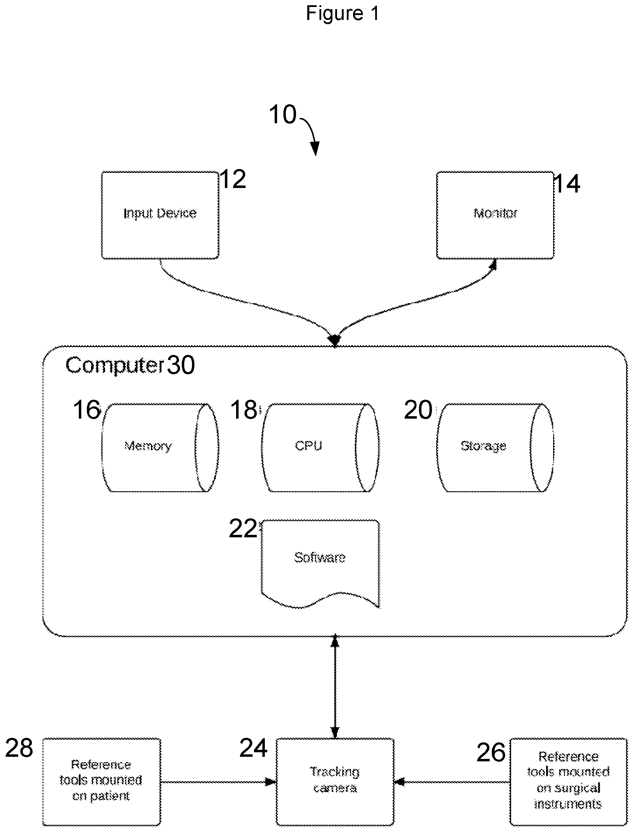

Fig. 1 illustrates the main components of a surgical navigation system 10 which can also serve pre-operative implant planning. System 10 includes an input device 12, a display device 14, such as a monitor, and a computer system 30 (or any type of suitable processing system having a memory unit 16, a CPU 18 and a storage unit 20) which runs software application(s) 22. At least one tracking device 24, such as a suitable camera, cameras or stereoscopic camera(s), exchanges data with the computer system 30 so as to record positional data from patient mounted reference tools 28 and surgical instrument mounted reference tools 26. The at least one tracking device 24 can be any type of device that can sense the position of a reference marker or tool. For example, a tracking device might use electromagnetic radiation, such as visible light, infra-red radiation, or ultraviolet radiation, to sense the position of a reference marker or reference tool. Known tracking devices and reference markers can be used and are generally available.

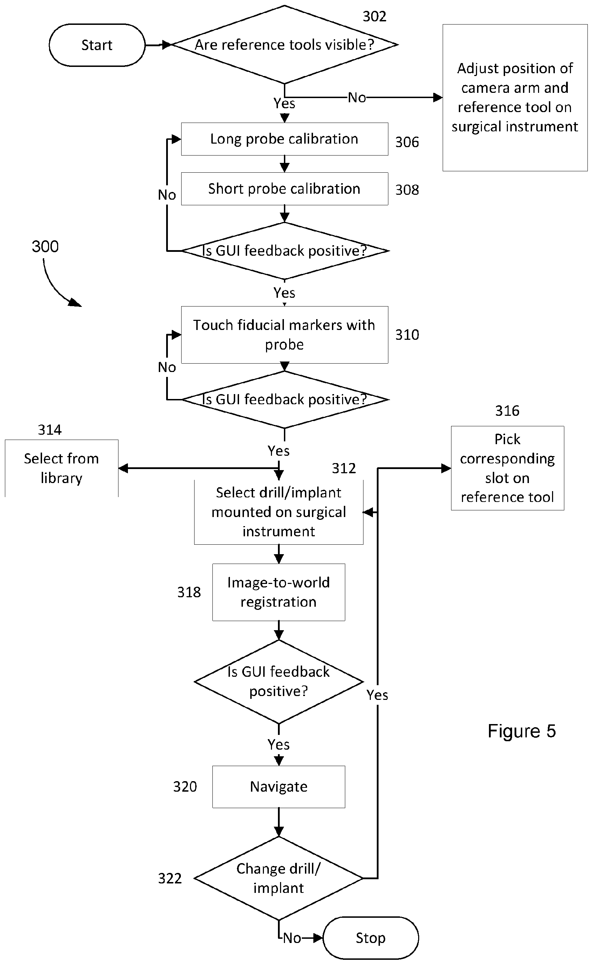

When interrogated by computer system 30, through a dedicated operation in the controlling software application, for example as per the steps in Fig. 5, the at least one tracking device 24 reads the real-world physical position and axial orientation of the reference tools 26 and 28, which are provided with one or more reference markers. The real-world physical position and axial orientation of one or more surgical instruments are then transformed into spatial coordinates relative to the same virtual environment where one or more images of the patient's anatomical structures are represented. This action allows a surgeon to visualize the precise position and axial orientation of the surgical instrument(s) during the surgical operation. Preferably, in the same display window of the software application that presents information on display device 14 to the user, the position and/or axial orientation of the tracked surgical instrument(s) is presented together with one or more pre-planned implant position.

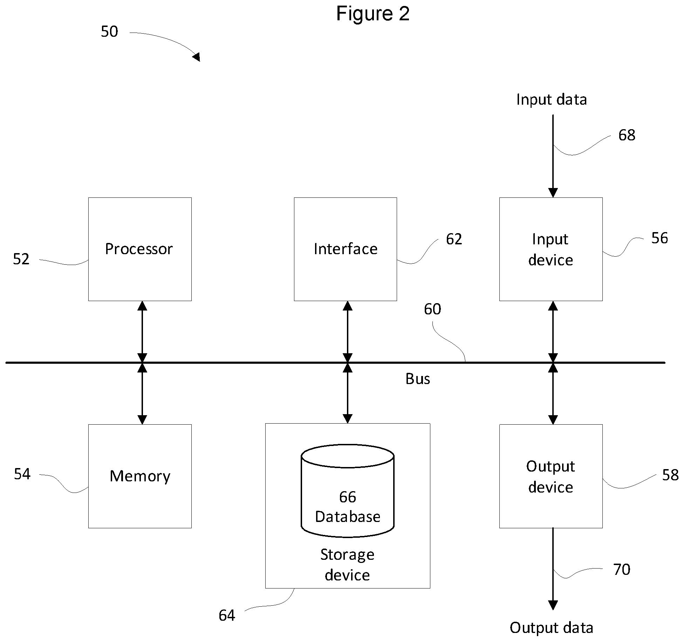

The processing system 50 which is shown in Fig. 2 is an example of computer system 30. In particular, processing system 50 generally includes at least one processor 52, or processing unit or plurality of processors, memory 54, at least one input device 56 and at least one output device 58, coupled together via a bus or group of buses 60. In certain embodiments, input device 56 and output device 58 could be the same device. An interface 62 can also be provided for coupling the processing system 50 to one or more peripheral devices, for example interface 62 could be used to connect one or more tracking cameras 24. At least one storage device 64 which houses at least one database 66 can also be provided, either locally or remotely. The memory 54 can be any form of memory device, for example, volatile or non-volatile memory, solid state storage devices, magnetic devices, etc. The processor 52 could include more than one distinct processing device, for example to handle different functions within the processing system 50.

Input device 56 could be a data receiver or antenna such as a modem or wireless data adaptor, data acquisition card, etc., and receive input data 68, or could be, for example, a keyboard, touch screen, a pointer device such as a pen-like device or a mouse. Input data 68 could come from different sources, for example keyboard instructions in conjunction with data received via a network. Output device 58 produces or generates output data 70 and can include, for example, a display device or monitor in which case output data 70 is visual, a printer in which case output data 70 is printed, a port for example a USB port, a peripheral component adaptor, a data transmitter or antenna such as a modem or wireless network adaptor, etc. Output data 70 could be distinct and derived from different output devices, for example a visual display on a monitor in conjunction with data transmitted to a network. A user, such as a surgeon, could view data output, or a visual representation of the data output, on, for example, a display device. The storage device 64 can be any form of data or information storage means, for example, volatile or non-volatile memory, solid state storage devices, magnetic devices, etc., and could reside locally or remotely to the other components of the processing system.

In use, processing system 50 can also be adapted to allow data or information to be stored in and/or retrieved from, via wired or wireless communication means, the at least one database 66. The interface 62 may allow wired and/or wireless communication between the processing unit 52 and peripheral components that may serve a specialised purpose, e.g. a tracking camera. The processor 52, running the software application or module thereof, receives instructions as input data 68 via input device 56 and can display processed results or other output to a user by utilising output device 58. More than one input device 56 and/or output device 58 can be provided. It should be appreciated that the processing system 50 may be in a variety of forms such as a digital device, computerized device, tablet device, terminal, client terminal, server, specialised hardware, or the like.

The processing system 50 may be a part of a networked communications system and could connect to network, for example the Internet, a LAN or a WAN. Input data 68 and output data 70 could be communicated to other devices via the network, thus facilitating processing system 50 to be operated remotely. Hence, system 10 could also be operated remotely. For example, robotic apparatus can be used to physically manipulate surgical instruments thereby allowing a surgeon to be located remotely to a patient while the surgeon can view displayed images and surgical instrument positions and axial orientations and remotely control the robotic apparatus.

Processing system 50 can also retrieve information or data, such as reference tool data or template data, from a local or remote information source, which can include a local database or memory, a server, or any type of terminal, that may be associated with one or more storage devices that are able to store information or data, for example in one or more databases residing on a storage device. The processing system 50 can be adapted to communicate with other terminals, for example further processing systems, by sending and receiving data to and from a network, thereby facilitating possible communication with other components of a networked communications system, such as the Internet, LAN or WAN.

### Computer-Implemented Method

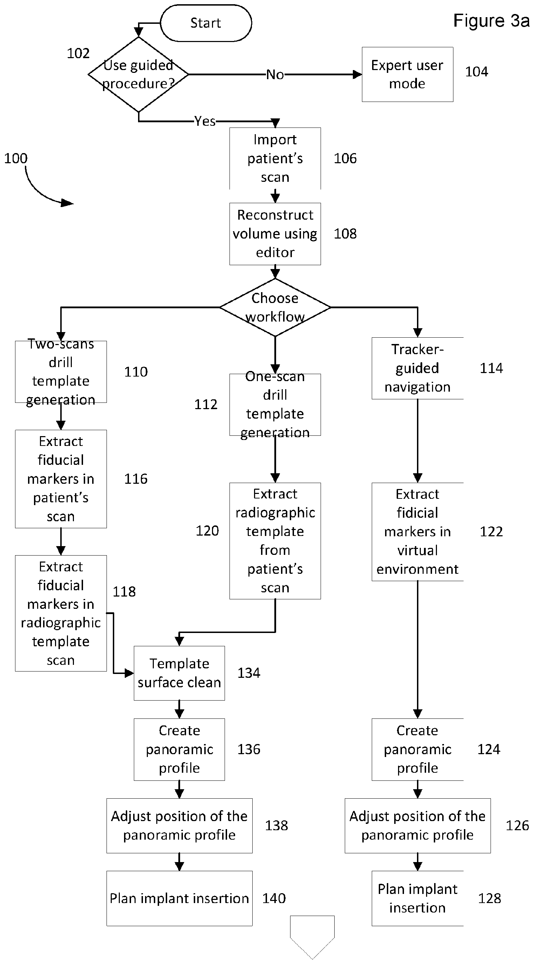

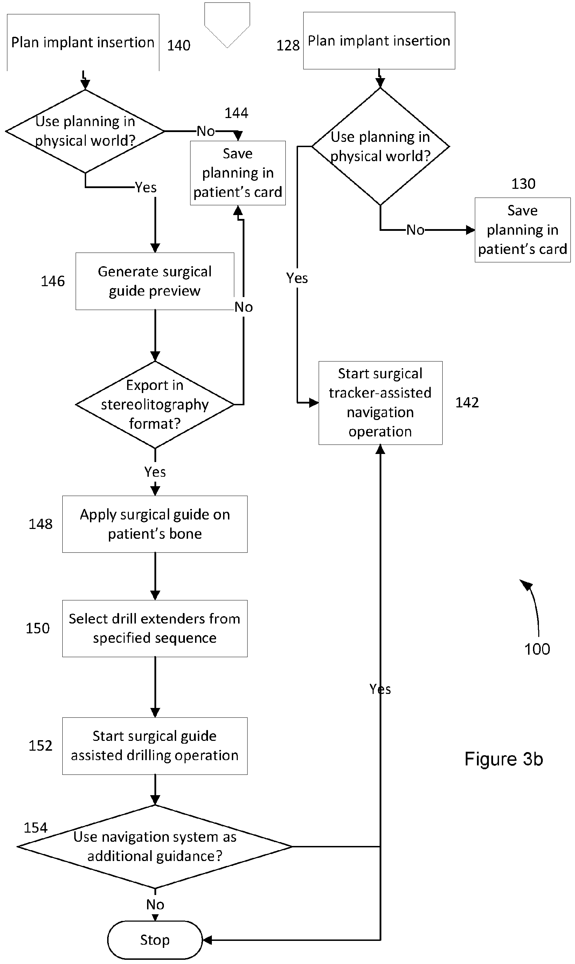

Computer-implemented methods, effected by a software application or computer program, which may be implemented as one or more software components, modules or procedures, are illustrated in Figs. 3a to 5. Firstly referring to Figs. 3a and 3b, the software application can run as a computer-implemented method 100 providing a guided procedure, that offers surgical planning. The user, which may be for example a surgeon or other type of medical practitioner or specialist, is asked at step 102 if he or she wishes to use a guided procedure. The guided procedure requires the user to import a patient's scan as step 106. If not selected, at step 104 the user can enter an expert user mode. At step 108, a volume is reconstructed according to the patient's tomographic data, which becomes the main datum for two guided surgery workflows.

The user can design a drill guide template for use in later surgery in either of two ways: a two-scans procedure and a one-scan procedure. The main difference between these two methods is the method used to digitize a radiographic template which is used as basis for the design of the surgical template.

In the one-scan procedure 112, a radiographic template is isolated from the patient's bone via the high radio-opacity contrast of a radiopaque material within a dedicated operation in the software application. At step 120, the radiographic template is extracted from a patient's scan. In the two-scans procedure 110, the radiographic template is identified in two consecutive scan steps 116, 118 via a software operation which allows a digital marking of radiopaque fiducial markers.

Once a drill guide template has been identified against the near anatomical structures, its digital surface can be edited at step 134 in the software application. In one embodiment, the main compound views offered to the user (e.g. the surgeon) in the software application allow implant surgery planning, and subsequently navigation, to be performed. These views are built according to the patient's panoramic profile created by the surgeon via a dedicated operation at step 136.

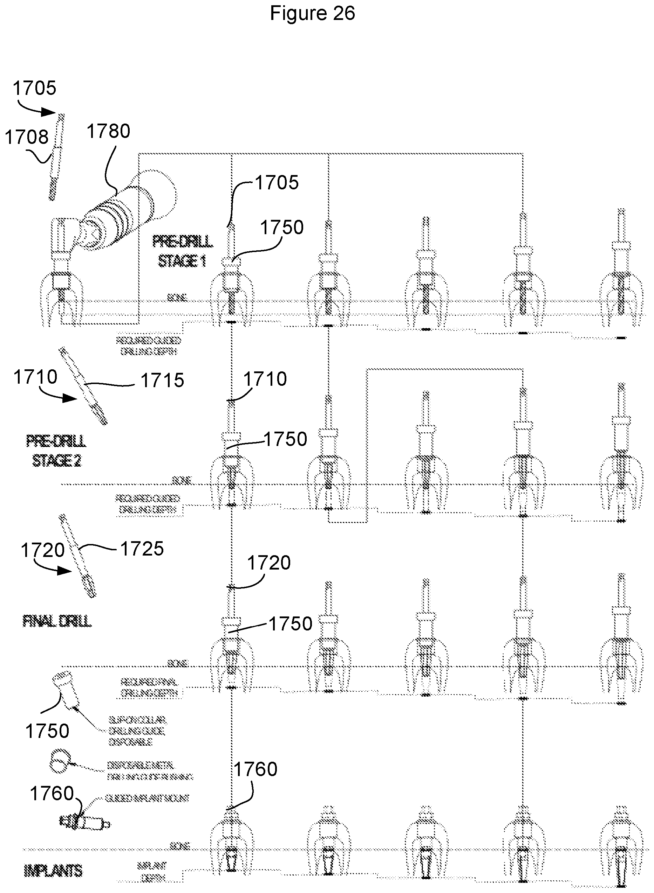

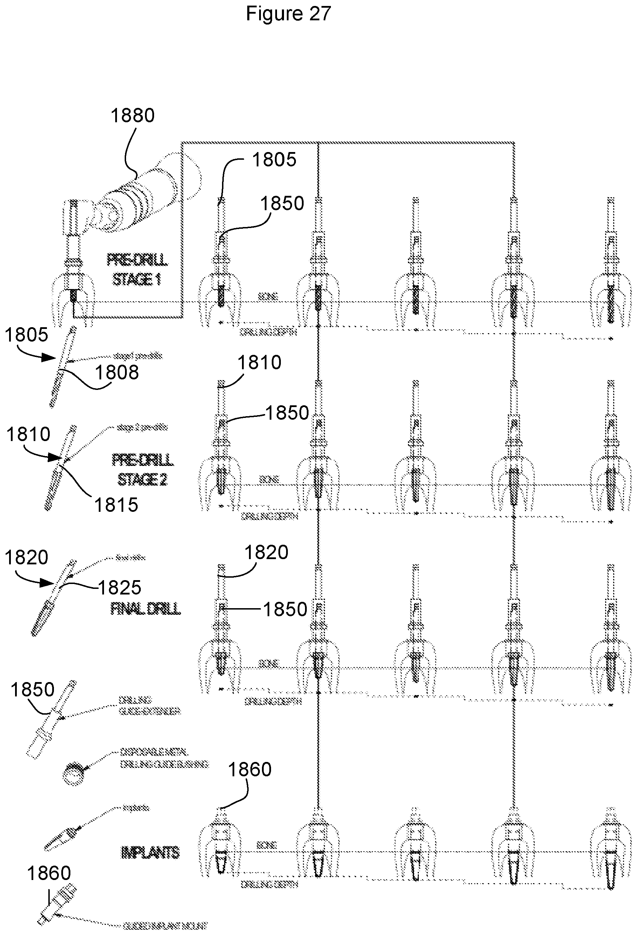

In order to simulate the insertion of implants, the user selects the implants from a library provided in a database or creates a customized implant via a dedicated software module and picks a desired start point and end point in a main slice view, which provides step 140 for planning insertion of implants. When all the implants have been inserted the user can save the study at step 144, for example as part of a patient's digital records, or proceed to step 146 for the generation of the parameters needed to design the surgical guide template. In this process the software application provides a combination of the positioned implants and their relative socketing (or locations) into the radiographic template. The socketing parameters are designed to accommodate the drilling procedure via the use of extended shaft drills or extenders as described respectively in Fig. 26 and Fig. 27. The design of the components needed to realize the surgical guide template can be sent to production in a desired format, for example proprietary/open CAD file.

In subsequent surgical use, procedure 100 progresses to step 148 where a surgeon applies the surgical guide, that has been physically produced from the radiographic guide or virtual surgical guide, to the desired position on the bone of a patient. At step 150, the surgeon then selects an appropriate drill extender(s) from a specified set presented to the surgeon by the software application. At step 152, the surgeon can then start a drilling operation on the patient using the surgical guide and the selected drill or drill extenders, thus providing a Computer Assisted Surgery procedure.

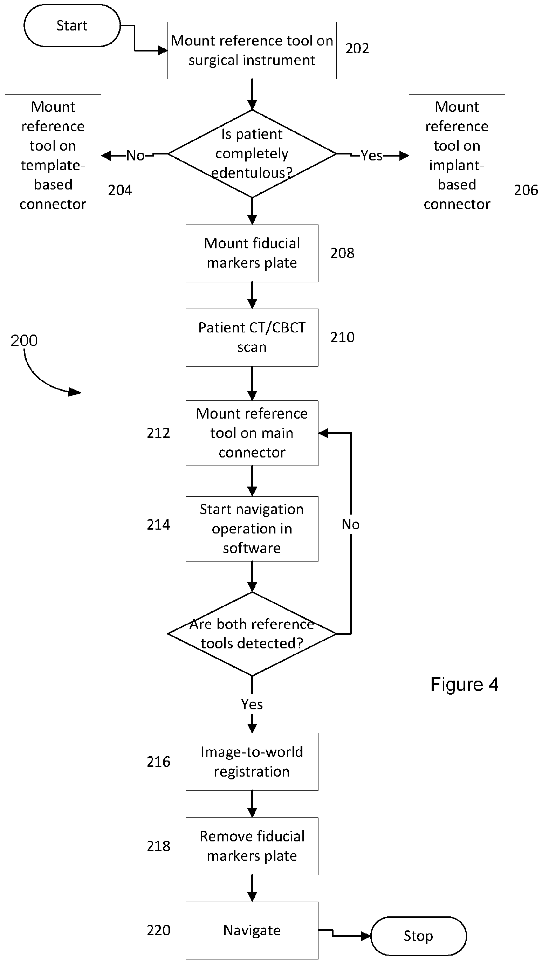

At step 154, the surgeon is provided with the option by the software application as to whether or not the navigation system should be used for additional guidance during surgery. If the surgeon (or other user) selects this option at step 154, then the software application, at step 142, starts a surgical tracker-assisted navigation operation (for example see Fig. 4 and Fig. 5).

Another Computer Assisted surgery option which the user/surgeon can pursue in order to maximise the accuracy of implant insertion is the use of a navigation system which tracks the surgical instrument(s) in use within the pre-operative virtual planning procedure, as illustrated as step 114. If this tracker-guided navigation procedure 114 is selected, then at step 122 fiducial markers are extracted in a virtual environment. At step 124, the software application allows the user to create a panoramic profile, and at step 126, the position of the panoramic profile can be adjusted using the software application. At step 128, insertion of implants is then planned by the surgeon using the software application. The user or surgeon is then prompted to select if the planned implants are to be physically used; if yes, then at step 142 the surgical tracking-assisted navigation operation is started; if no, then at step 130 the virtual surgical guide with planned implants can be saved as a data file, for example in a patient's card or record.

Referring to Fig. 4, there is illustrated a method 200 providing a navigation mode implemented by the software application. In this method, at step 202 a reference marker tool (or holder), as will be described later, is mounted on a surgical instrument to be used in a surgical procedure. In the non-limiting example of oral implantology, if the patient is completely edentulous then at step 206 the reference tool is physically mounted on an implant-based connector. Otherwise, if the patient is not completely edentulous, then at step 204 the reference tool is mounted on a template-based connector. Depending on the particular surgical procedure, different forms or types of reference tool can be mounted on different forms or types of connectors. Depending on the circumstances, a connector may be template-based or implant-based or some other form of connector.

At step 208, a fiducial markers plate is mounted on the connector. At step 210, the patient is scanned, for example using a CT/CBCT scan or any other type of appropriate 3D scan to produce required 3D representations or images of the patient's anatomy. At step 212, a further or second reference tool is mounted on the connector.

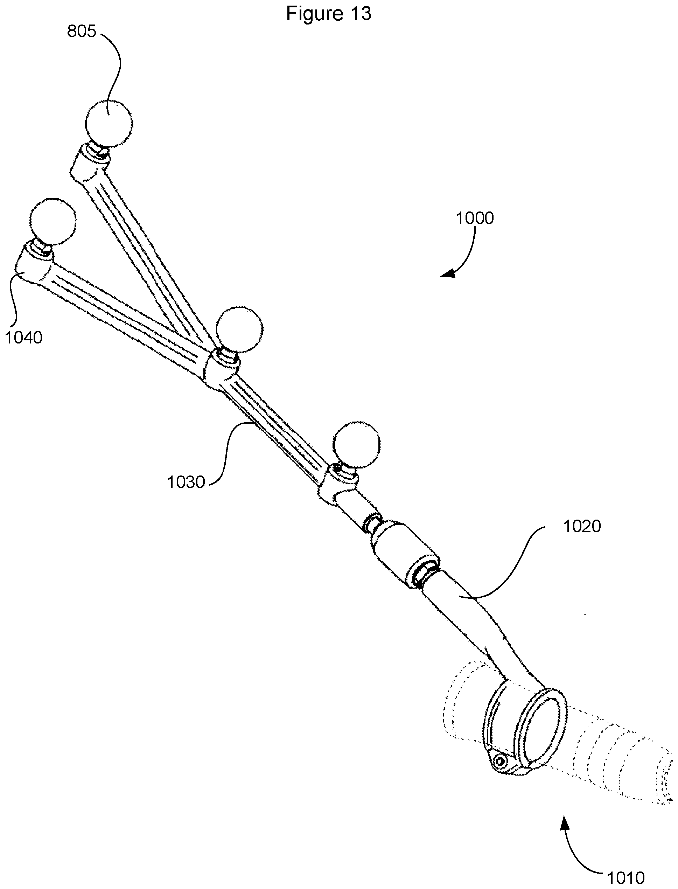

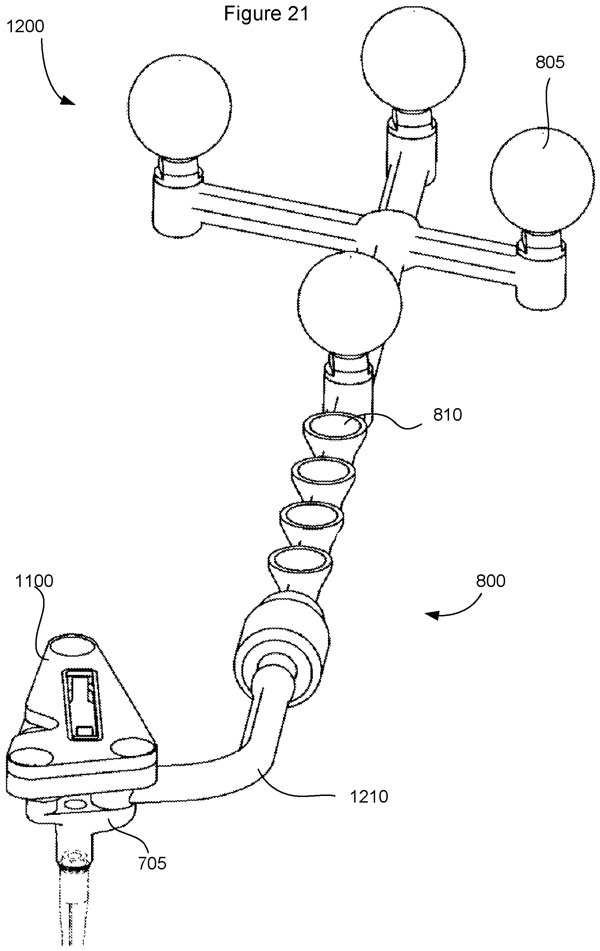

Thus, there is now provided a first reference tool mounted on a surgical instrument and a second reference tool mounted on the connector which also holds or supports a fiducial markers component, e.g. a fiducial markers plate. Physical examples are described later, for example in Fig. 13 and Fig. 21.

At step 214, a navigation operation is started in the software application. A check is made to see if both reference tools have been detected by the navigation system, and if so, then at step 216 an "image-to-world" registration is performed by the software application. At step 218, the fiducial markers plate can be removed, and at step 220 the surgeon is then able to navigate using the software application, meaning that a precise visual or virtual image of the physical real-world position and axial orientation of the surgical instrument in accurate relation to the patient's anatomy, provided by the scan of the patient, is provided by the software application.

Referring to Fig. 5, there is illustrated a method 300 for aspects of the surgical procedure assisted by the surgical navigation system. At step 302, the software application checks if the reference tools are visible. As discussed in relation to Fig. 4, it is preferred to provide a first reference tool on a surgical instrument and a second reference tool on a connector. It should be realised that more than two reference tools can be used.

If the reference tools are not visible, then at step 304 the user is prompted to check settings such as adjusting the position of one or more cameras or camera supports, and the reference tool mounted on the surgical instrument. If the reference tools are properly visible and detected by the system, then prior to use on a patient, a calibration process can be performed. At step 306 calibration using a long calibration prober is performed, and then at step 308 calibration using a short calibration prober is performed. This calibration procedure might use a calibration tool 905 for facilitating pivoting the probe. Alternatively the probe position could be determined by reference to a socket 830 positioned on the reference tool. If the Graphical User Interface (GUI) indicates to the user that calibration is accurate, that is positive feedback is provided, then at step 310 the fiducial markers are touched with the end of the short calibration prober. Again if the calibration feedback is positive, then the method progresses to step 312 where the software application requests a selection of the drill type and/or the implant type mounted on the surgical instrument. At step 314, this selection may be from a button type list of options provided to the user, or at step 316 this selection may be from use of a corresponding slot on the reference tool. At step 318, "image-to-world" registration is then performed which maps virtual images to the physical world. Again, a check can be made if the GUI is indicating that feedback is positive. If no, the calibration procedure can be re-initiated from the start. If yes, method 300 can progress to step 320, where the user is able to navigate using the system, that is the surgical instrument should be properly tracked in the physical world and precise images of position and axial orientation of the surgical instrument visually displayed to the user. Should the user wish to change the drill or implant, then at step 322 an option is provided by the software application to return to step 312 so that the user can re-select the drill or implant physically mounted on the surgical instrument.

Thus, in one embodiment there is provided a computer-implemented method for surgical navigation or planning. The method comprising: obtaining, by at least one computer system, a three-dimensional (3D) anatomical representation of part of a patient; and producing, by the at least one computer system, at least one two-dimensional volume slice view of the three-dimensional anatomical representation, the at least one two-dimensional volume slice view based on a physical position or axial orientation of a surgical instrument, or a virtual position or axial orientation of a surgical implant. The method then involves displaying, by a display device, the at least one two-dimensional volume slice view to a user; and further, updating, by the at least one computer system, the displayed at least one two-dimensional volume slice view based on movement of the physical position or axial orientation of the surgical instrument during surgical navigation, or by movement of the virtual position or axial orientation of the surgical implant during surgical planning.

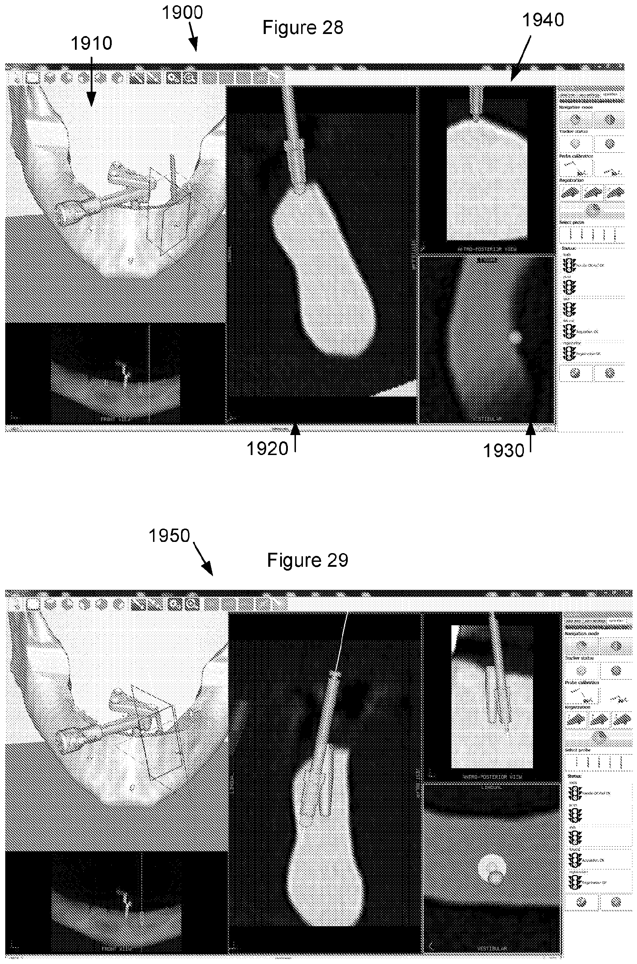

The display device is used to simultaneously display a view of the three-dimensional anatomical representation and the at least one two-dimensional volume slice view to the user, for example as shown in Figs. 28 and 29. In another example, the at least one computer system is able to produce two or more two-dimensional volume slice views, and the display device is able to simultaneously display the two or more two-dimensional volume slice views to the user, again for example as shown in Figs. 28 and 29.

In a particular but non-limiting example, the tracking algorithm used to track reference markers can utilise the NDI Vicra/Polarisfirmware APIs, which are able to provide as output the 3D positions of each reference tool with respect to the camera's reference system. Calibration, registration and live tracking can then be performed by applying further algorithms to the output 3D position data. For example, the 3D positions are transformed into new coordinates in the virtual environment where the tomographic scan's volume is present. The transformation is made possible via the computation of a matrix which is calculated according to the known general Iterative Closest Point registration algorithm. Preferably, though not necessarily, the calibration is instead completely performed by calculating the average physical point of the drill tip from a collection of frames, then computing the axis of the surgical tool after a second frames collection. Live tracking is achieved by combining the information of the calibration with the registration matrix and the current positions read by the camera's firmware. The on-the-fly calculation is preferably carried on using multiple threads within the operating system.

The computer-implemented methods can be implemented using any desired computer programming language, and may be implemented as a single software package or as a collection of modules, procedures or components. The software application can run locally on the processing system, or could run on a remote server and the local processing system could be a thin client or simply a display and input devices. The user can interact with the software application by commonly known means, for example using a keyboard, mouse or pointer device, and/or by using a touch screen display. The at least one computer is able to transform the physical position and axial orientation of the at least one reference tool into spatial coordinates relative to the three-dimensional anatomical representation of part of the patient.

### Reference Tools, Connectors and Markers

In order for the navigation system to accurately track and display surgical instruments in their correct position and axial orientation, the tracking device 24, such as a stereoscopic camera(s), must identify the markers physically mounted both on the patient's anatomical structure and physically mounted on the surgical instrument to be tracked (for example using the steps of Fig. 4).

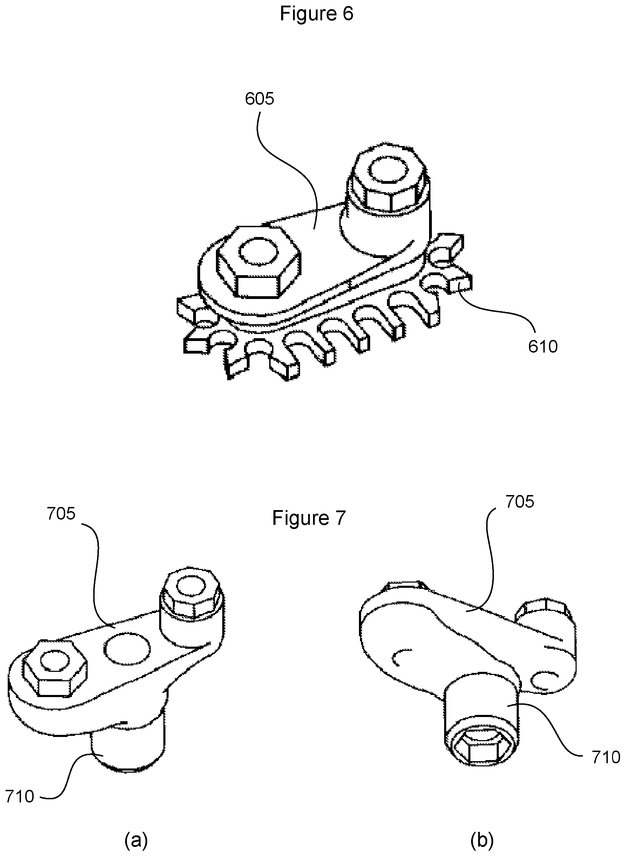

This requires that the markers are fixed in specific configurations. The reference tool which supports the markers is mounted on a connector. In case the patient is completely edentulous the connector is mounted on an implant using an implant socket, as shown in Fig. 7. If the patient is partially edentulous the connector can be fixed to a radiographic template, as shown in Fig. 6, while the radiographic template is still setting.

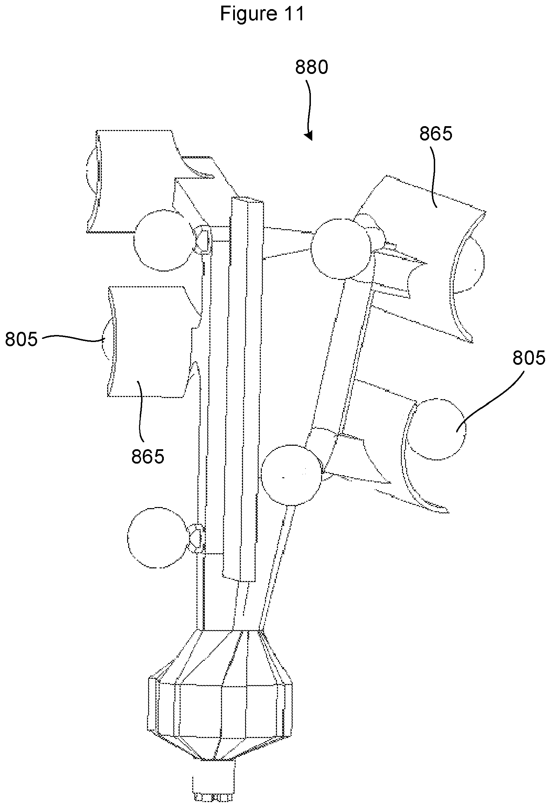

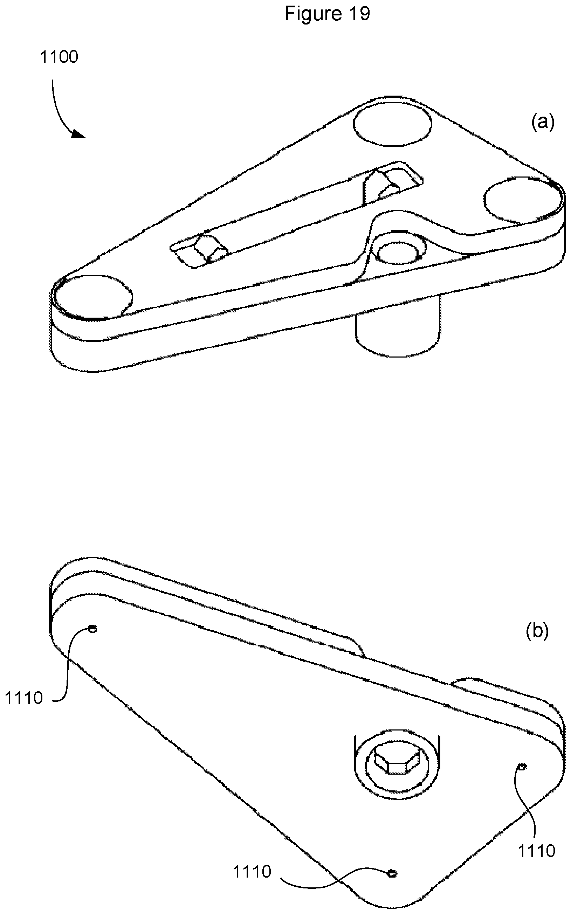

Turning to Fig. 21, before the patient undergoes a tomographic scan, the surgeon/user must mount the fiducial markers plate (see Figs. 19(a) and 11(b)) on the connector (see Figs. 6 or 7). The fiducial markers plate provides for the image-to-world registration during the navigation setup. Once a tomographic scan of the patient has been taken, the user can load the digital data within the guided procedure and start using the software application in navigation mode.

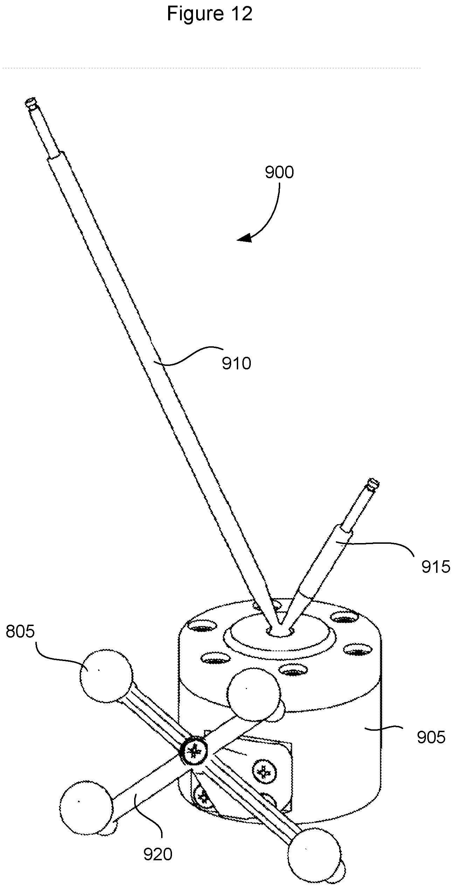

The calibration procedure required by the navigation system allows the user to track both tip and axis of one of the multiple drills or implants from the software application's library, which can be an internal library or a remote library. In case the calibration of the tip is performed using a recommended fixed-length probe, switching from one instrument tip to the another does not require a re-calibration. The switch is achieved by selecting a software button/option corresponding to the specific drill/implant from the library which the user is about to mount on the surgical instrument, or by touching with the prober a specific socket manufactured on the reference tool . Calibration of the surgical instrument could be achieved in two steps with the use of the calibration device (see Fig. 12) and respectively a long and a short tip probers. The successful completion of the calibration steps is reported within the navigation panel in the software interface and allows the accurate tracking of both the ultimate tip used and the axis of the surgical instrument.

The software application creates a digital 3D representation of the reconstructed volume from the patient's scan in which the fiducial markers plate is visible (see Fig. 19). A dedicated operation within the software allows a semi-automated recognition of the markers of the fiducial markers plate. By using a dedicated operation and physically probing the position of the fiducial markers, the user allows the software application to register the position of the tracked surgical instrument with respect to the patient's real-world anatomy.

Specific, but non-limiting, examples of the reference tools, markers, connectors, plates and associated parts or components, are now described. Referring to Fig. 6, there is illustrated a connector 605 which is fixed to or mounted on a radiographic template 610. This type of main connector arrangement can be used if a patient is partially edentulous. Referring to Fig. 7, another type of connector 705 can be fixed to or mounted on an implant using implant socket 710 (or termed a protrusion or adapter). This type of main connector arrangement can be used for completely edentulous patients.

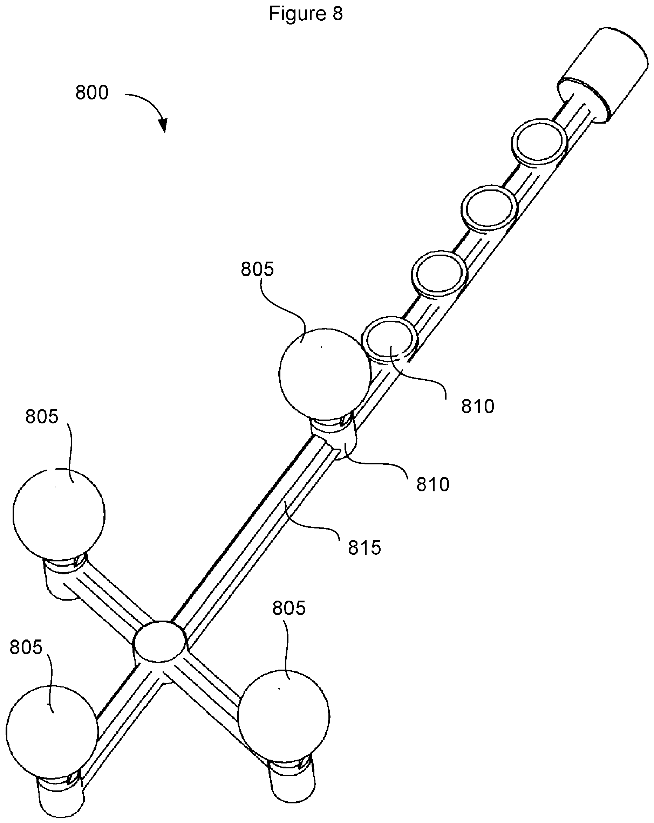

Referring to Fig. 8 there is illustrated a reference markers tool (also referred to as a reference markers holder) 800 that can be attached to a connector, such as connector 605 or connector 705. The shape and/or geometry of reference markers tool 800 can be significantly varied and the specific tool shape and type illustrated is for example only. Reference markers 805, which are preferably optical reference markers, and for example spherical shaped reference markers, can be attached to tool 800. Reference markers 805 are detected by the tracking device 24, for example being one or more tracking cameras. A series of sockets 810, or other forms of attachment means or mechanisms, are provided along a longitudinal arm 815 of tool 800. A reference marker 805 can be positioned and held in a socket 810. Reference markers 805 can be held in sockets 810 by a variety of means, by example by threaded engagement or an abutting fit. Also, tool 800 might be provided with at least some, or all, reference markers 805 permanently fixed to tool 800 so that sockets 810 need not be provided for variable marker locations.

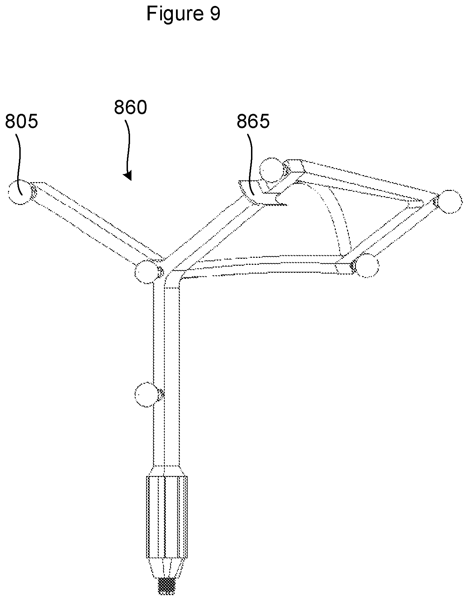

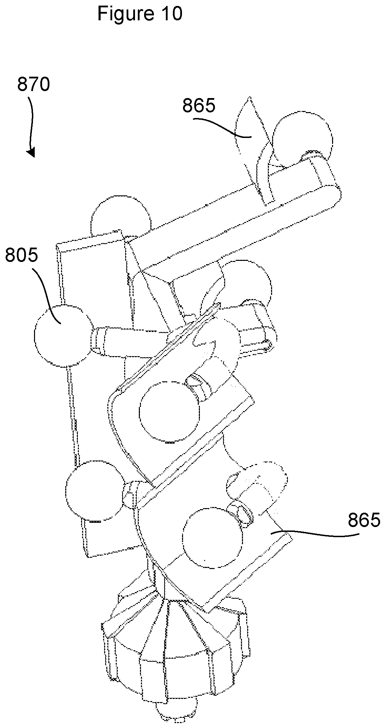

In other examples the reference tool is a multi-faced reference tool 860, 870 or 880, examples of which are provided in Figs. 9, 10 and 11. A reference tool may include more than four markers. In the case that more than four markers are used, the markers 805 can be fixed in such a way that their relative geometrical centres lay on one, two or three different planes. Optionally, one or more shields 865 can be provided are desired locations so that a marker is only visible to the tracking device from a range of angles. This configuration gives the reference tools 860, 870 and 880 a multi-faced geometry. The aim is to promote the visibility of the reference tools 860, 870 and 880 even when they form a wide angle with respect to the tracking device. In this way the surgeon is able to operate on the patient from different angles in a more comfortable fashion.

Referring to Fig. 12 there is illustrated a calibration device 900 that is used for calibrating a surgical instrument so that respectively tip and axis of the surgical cutting bit can be virtualised, or otherwise to improve positional accuracy. Calibration device 900 includes a body 905 which supports a long calibration prober 910 or a short calibration prober 915. In the example illustrated, long calibration prober 910 or short calibration prober 915 are attached to body 905 via a tight fit moveable joint so that the position of the probers 910, 915 can be moved. For convenience, both long calibration prober 910 and short calibration prober 915 are illustrated together, however, in the preferred embodiment only long calibration prober 910 or short calibration prober 915 is used. Cross-arms 920 are attached to body 905 and hold further reference markers 805. These reference markers can be used to determine the position and axial orientation in the physical world of calibration device 900, and especially the physical positions of the end points of probers 910, 915, to which instruments or devices can be touched as part of the calibration process.

Referring to Fig. 13, there is illustrated a reference tool 1000 that attaches to a handle of a surgical instrument 1010, with an example handle shape illustrated. Attachment to a variety of surgical or diagnostical instruments is possible, such as a drill tool or an implant tool, for example by clamping to a handle portion of a surgical instrument. Reference tool 1000 includes a handle support 1020 that is used to physically attach the reference tool 1000 to the surgical instrument 1010. A variety of attachment means can be used, for example clamping, screwing, bolting, etc., handle support 1020 to surgical instrument 1010. It is also possible that the reference tool 1000 could be integrated with a surgical instrument. Support frame or arms 1030 attach to handle support 1020, and support frame or arms 1030 allow reference markers 805 to be attached to or mounted thereon. As previously, reference markers 805 may attach to or be received by sockets 1040 provided as part of support arm or frame 1030, or alternatively reference markers 805 may be permanently attached to support frame or arms 1030. It should also be appreciated that, if desired, any of the reference markers 805 used on the various tools could be different reference markers, for example different shapes, different sizes, different dimensions and/or different materials. Although it is an option, it is not necessary that all reference markers be identical or similar.

Preferably reference tool 1000 is a rigid tool which supports optical reference markers 805, and is attached to the surgical instrument via rigid handle support 1020 (or termed an arm or bracket). Different shaped handle supports can be designed to facilitate use by left-handed or right-handed practitioners. The configuration of the reference markers 805 may, but need not necessarily, be as shown in Fig. 13, and is designed to balance tracking accuracy and user comfort during implant surgery. The attachment of the rigid body supporting the reference markers to the handle support can be designed as a ball joint so that the user has three degrees of freedom in positioning the reference markers structure and therefore enhancing visibility by the camera.

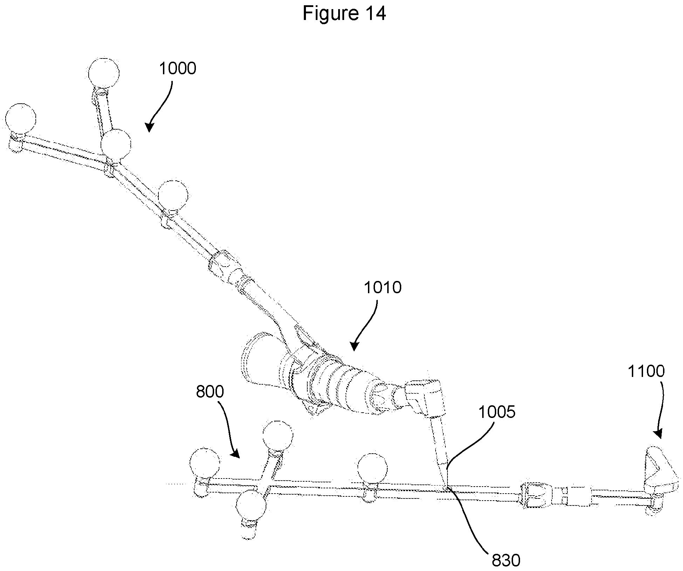

Alternatively or additionally, a one-touch calibration procedure can be provided, which is represented in Fig. 14. The one-touch procedure serves the purpose of identifying the drill tip 1005 and the axis of the drill tip/implant mounted on the surgical instrument 1010. Correct information on the position of the drill tip and the axis of the drill tip can be translated into an accurate representation of the surgical instrument 1010 within the surgical navigation software.

In the one-touch calibration procedure, the user touches a physical point 830 on the reference tool 800 whether before or after tool 800 is mounted on a patient. This physical point 830 can be identified by engraving a dimple or recess on the reference tool 800 in order to help locating the tip 1005 into alignment with the physical point 830. Differently to the pivoting calibration procedure presented in Fig. 12, the one-touch calibration procedure is a more comfortable solution for the insertion of maxillary implants since in this type of surgery the surgical instrument's tip is oriented upwards.

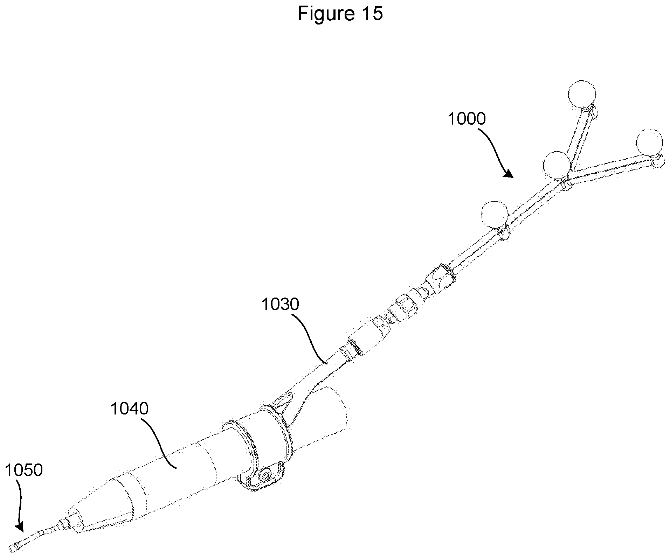

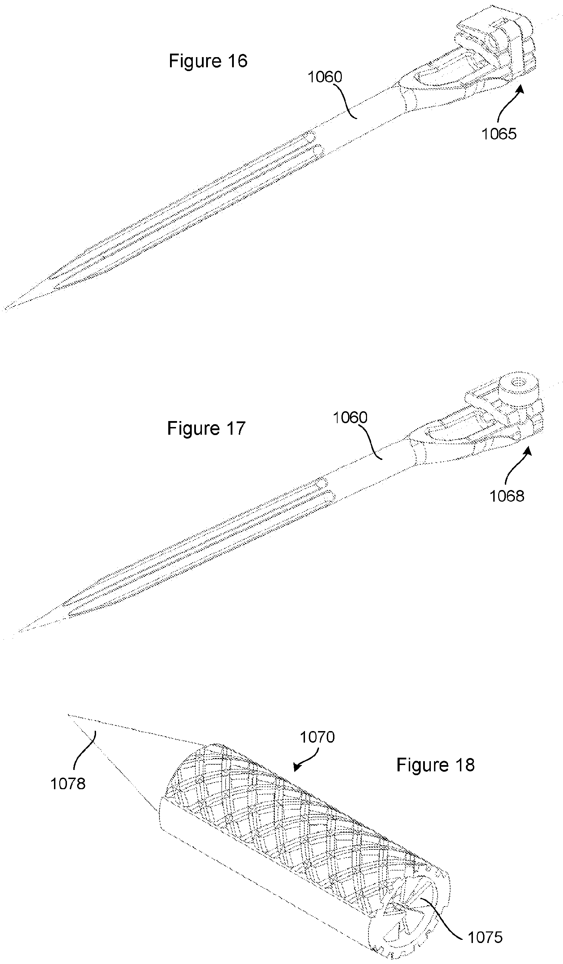

Now discussed are various embodiments having application to piezo-electric surgery with reference to Fig. 15. These embodiments widen the range of reference tool supports in order to include surgical instruments used for piezo-electric surgery. The use of the navigation system with piezo-electric surgical procedures, whether or not they are osteotomy of bone preparation for implant placement, includes the following three aspects.
(1) A reference tool support 1030 designed to hold a generic reference tool 1000 and clamp to a piezo-electric surgical handpiece 1040.
(2) A sterilisable and/or a disposable version of a dedicated calibration tool which allows the user to identify the axis of the piezo-electric tip 1050 and then transfer this information to the surgical navigation software.
   a. Sterilisable version - a sterilisable piezo-electric calibration tool 1060 is illustrated in Figs. 16 and 17 and includes an elongated straight tip which is anchored to the piezo-electric surgical tip 1050 and which is coincidental with the axis of the surgical tip 1050. The instrument's design allows for a recessed area which can accommodate any sort of piezo-electric surgical tip. The instrument's design includes a clamping mechanism 1065 using a hinged clamp, or an alternate clamping mechanism 1068 using a rotatable tightening bolt, which locks the calibration tool 1060 to the surgical tip 1050.
   b. Disposable version - a disposable piezo-electric calibration tool 1070 is illustrated in Fig. 18 and includes a cylindrical structure made of biocompatible material with a series of internal teeth 1075 designed to hold a surgical drill tip 1050 in a stable position. Once the surgical drill tip 1050 is inserted into the disposable piezo-electric calibration tool 1070 the axis of the most distal part of the surgical tip 1050 is coincidental to the symmetry axis of the cylindrical structure of the calibration tool 1070. The tip 1078 of calibration tool 1070 is then used for the one-touch calibration procedure or the pivot calibration procedure.
(3) The calibration of the short tip of a surgical instrument (whether it is used for piezo-electric surgery or classic implant placement surgery) can be replaced by one-touch calibration or a pivoting calibration of the surgical drill tip which is to be used during the live tracking procedure.

Referring to Figs. 19(a) and 19(b) there is illustrated a fiducial markers plate 1100. The geometry/shape of plate 1100 can be varied and the triangular geometry illustrated is by way of example only. The fiducial markers plate 1100 includes conical socket guides for fiducial markers. Also provided are holes 1110 which can receive radiopaque material, which is an example of a fiducial marker.

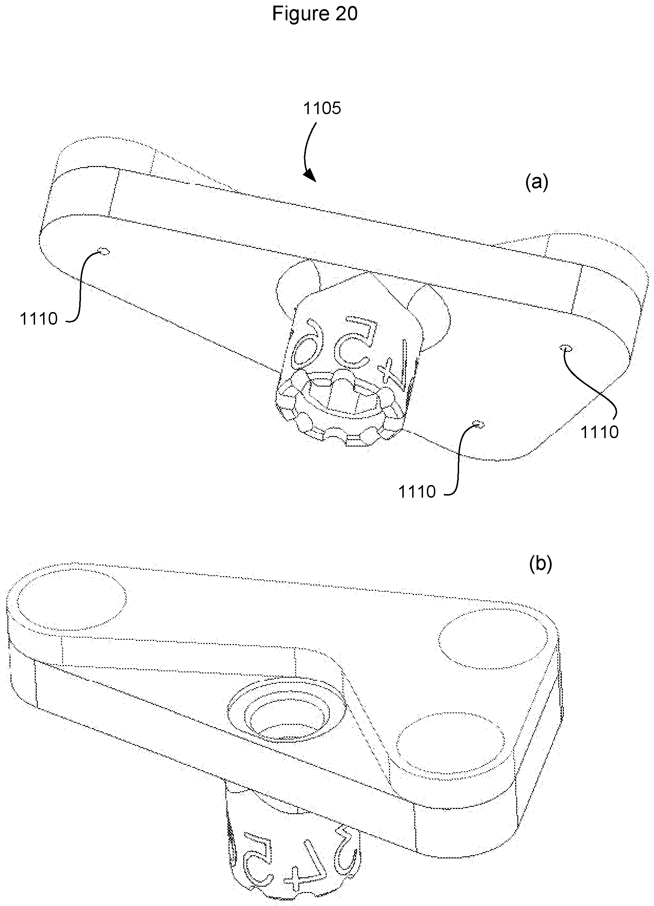

The fiducial markers plate 1100 is a rigid reference structure and is mounted on the patient to track a rigid region of the patient's anatomy, e.g. bone. The structure has to be able to preserve the relative position of reference markers tool 800 with respect to fiducial markers plate 1100 which holds radiopaque material providing fiducial markers which enables a determination of the physical position of the radiopaque markers with respect to the patient. The software application identifies and orders the fiducial markers (radiopaque material) from the patient's scan due to the known physical configuration on the fiducial markers plate 1100. Thus it is necessary that the position of the fiducial markers plate 1100, or more precisely the position of the fiducial markers themselves, relative to the patient's anatomy is unchanged between the scan of the patient and the image-to-world registration stage during the surgical procedure. Figs. 20(a) and 20(b) illustrate bottom and top views of a modified design of a fiducial markers plate 1105. The base of the fiducial markers plate may include an engraved scale useful to identify the mutual position of the plate with respect to the connector 705.

Referring to Fig. 21, there is illustrated an assembly 1200 utilising the reference markers tool (i.e. holder) 800, as previously described. Connector arm 1210 is used to connect reference markers tool (i.e. holder) 800 to the main connector, in this example being connector 705. Fiducial markers plate (also called a fiducial markers holder) 1100 attaches to connector 705. Connector 705 can be attached via socket 710 (equally a protrusion or adapter) to an implant in a patient. In an alternate version, connector 705 could be connector 605, or any other type of connector. The use of reflective reference markers, such as reference markers 805, can be complementary or alternative to the use of active markers of different geometry and shape, for example based on Light Emitting Diode (LED) technology.

By use of the reference tools 800, 1000 and the computer-implemented methods provided by the software application(s), the precise position and axial orientation of a surgical instrument can be determined in relation to a patient's anatomy, which in turn is known or can be determined in relation to fiducial markers plate/holder 1100 which includes radiopaque material.

The other Computer Assisted Surgery option which the user can pursue in order to maximize the accuracy of implant insertion involves use of the navigation system which tracks the one or more surgical instruments based on information obtained in the pre-operative virtual planning environment. In order for the navigation system to track and display the instruments in their correct position and axial orientation, the tracking device, such as a stereoscopic camera, must identify the optical reference markers mounted both on the patient's anatomical structure and on the surgical instrument to be tracked.

When the system is used for oral surgery, by way of example, and on a patient who is completely edentulous, the connector which supports the reference tool 800 is mounted on a standard implant (see Fig. 7). The shape of the connector is made to fit a common external hexagon attachment on the implant. Both the fiducial markers plate 1100 and the reference tool 800 are attached to the connector.

When the system is used for oral surgery, by way of example, and on a patient who is partially edentulous, the connector can be fixed to a radiographic template 610 (see Fig. 6) while the radiographic template is still setting.

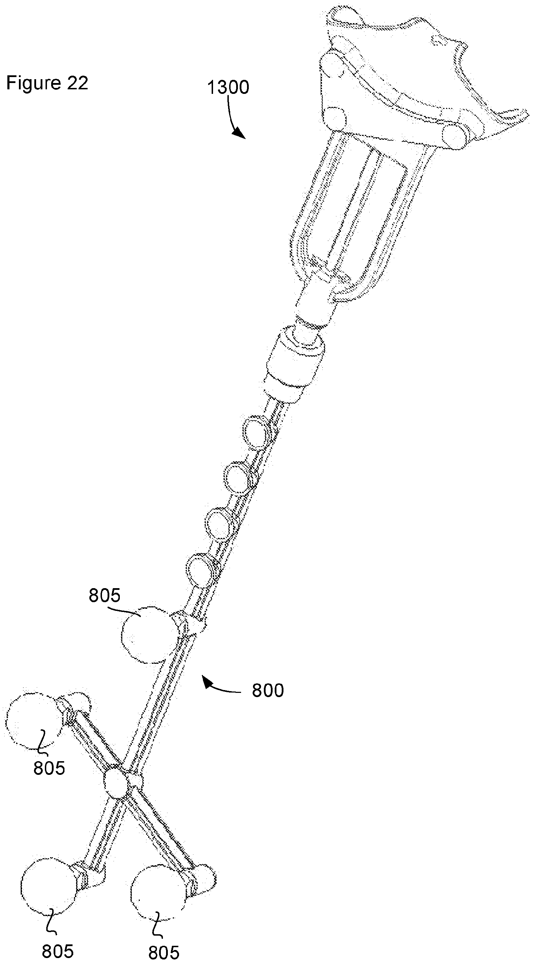

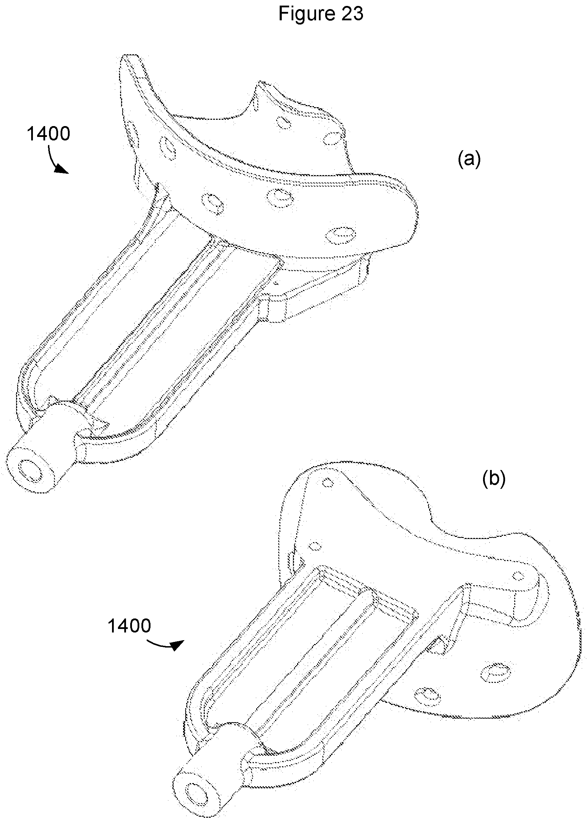

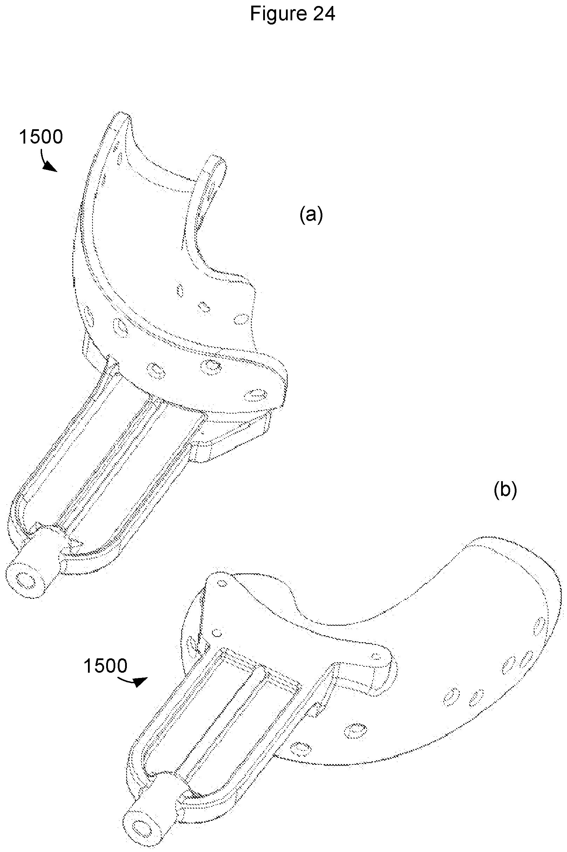

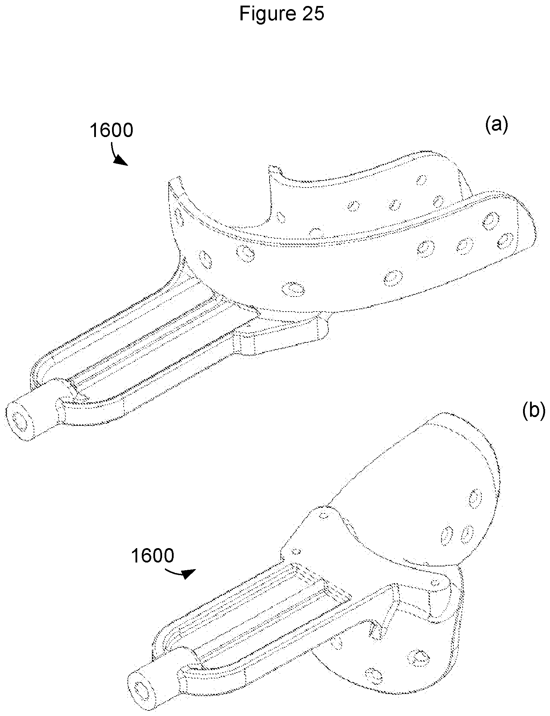

Alternatively, a different device termed a "fiducial tray" can be used, which is a type of anatomical adapter. Referring to Figs. 22 to 25, different examples of fiducial trays 1300, 1400, 1500 and 1600 are illustrated which are for oral surgery. A fiducial tray is used to adhere to the patient's anatomy before a scan through a fast-setting resin, and the fiducial tray can be re-located on the patient's anatomy with accuracy for the surgical procedure. The fiducial tray is connected to the reference tool 800 and the fiducial markers are embedded in the fiducial tray in a specific fixed configuration. Fiducial trays can be made to include trays to fit patients which are partially edentulous on one or multiple sides, for example as illustrated in Figs. 23, 24 and 25. Fig. 22 shows an example of how reference tool 800 can be mounted on, or connected to, a fiducial tray 1300. The connection may be using a mechanical joining that can be fixed, for example a hinged or ball and socket type arrangement that is variable but can be fixed or locked into position. The connection between the fiducial tray 1300 and the reference tool 800 is designed to preserve the mutual position of the reference markers 805 with respect to the fiducial markers (radiopaque material) during the calibration procedure. The components of the assembly must not change relative positions with respect to the patient's anatomy during a tomographic scan or during the surgical procedure. The patient's anatomy is used to preserve this position by using a setting resin provided between the fiducial tray and the patient's anatomy.

The various components described can be made from a variety of materials or composites. In particular, but non-limiting embodiments, the connectors 605, 705, the support frame or arms of reference markers tools 800, 1000, such as longitudinal arm 815, and the fiducial markers plate 1100 can be made of titanium or a titanium alloy. The long calibration prober 910, the short calibration prober 915, the body 905 and the cross-arms 920 of the calibration device 900 can be made of stainless steel. The reference markers 805 can be made of a reflective material such as reflective plastic. The fiducial trays 1300, 1400, 1500, 1600 can be made of a polymer material(s), such as poly(methyl methacrylate) (PMMA) which is a transparent thermoplastic (e.g. sold under the trade name Perspex™). The handle support 1020 can be made of titanium, a titanium alloy or a hard plastic.

### Drill Sets, Drill Bits and Surgical Guides

During the operating procedure, the surgeon applies the template onto the patient's bone verifying and locating it firmly. In order for the drill guide to achieve improved accuracy in the procedure, the surgeon is requested to insert a selected item from a set of drill bits into the socketed template. A set of drill bits and/or guides have been developed by the applicant for this purpose.

Example embodiments are not limited to use of surgical drill guides. More generally, in further example embodiments, there can be provided other types of surgical guides. Surgical guides can be drill guides, saw guides, alignment guides, etc.. The combination of live tracking with the use of surgical guides, such as a surgical drill guide, and the surgical instrument, such as the drill bit, can be assisted by embedding fiducial markers and a connector for the optical reference tool in the structure of the surgical guide. Such a structure would preserve its functionality as socketing guidance, for example for drilling, and offer the rigidity between anatomical structure and radiopaque and optical markers needed for the image-to-world registration during the navigation procedure. Following is discussed a specific example of a surgical guide being a surgical drill guide.

To-date, the most commonly used technique for mechanically guiding the drill bit into the template socket, and avoiding drilling at undesired depths, makes use of a separate set of bushings and drill stops to be used in conjunction with an ordinary known drill bit. Distinct to known procedures, the applicant has devised drill bits that serve both required functions: constraining the drilling axis; and stopping the drilling operation at the correct depth according to the virtual implant planning.

A drill set can include a separate tool which merges an extender and a drill stop. Alternatively, the drill stopping structural feature can be embedded in the drill bit. The drills are designed so that they can slide and fit into the template socket until the drill/implant reaches its final position inside the patient's anatomy. The drilling operation can be executed in conjunction with live tracking of the surgical instrument used for the formation of the bone socket in order to check the congruency of the physical procedure with respect to the pre-operative planning, as at step 154 of Fig. 3.

Referring to Fig. 26 and Fig. 27, there are illustrated example drill sets which include drill bits. Referring to Fig. 26, example drill bits 1705, 1710, 1720 have been improved to have an extended drill shaft 1708, 1715 and 1725, respectively. A drill set can also include a separate collar 1750, preferably a slip-on collar, which, when attached to or about the drill shaft 1708, 1715 or 1725, provides both extender and drill stop functionalities to drill bits 1705, 1710 and 1720 when used in a drill 1780. After the drilling procedure, implant mount 1760 can be inserted which will be at the correct implant depth into the bone.

Alternatively, the drill stopping structural feature is embedded in or integrally formed as part of a drill tip extender 1850 as shown in Fig. 27. Referring to Fig. 27, example drill bits 1805, 1810, 1820 are shown. A drill set can also include the drilling guide extender 1850, which, when attached to, about or over the drill shaft 1808, 1815 or 1825, provides both extender and drill stop functionalities to drill bits 1805, 1810 and 1820 when used in a drill 1780. After the drilling procedure, implant mount 1860 can be inserted which will be at the correct implant depth into the bone.

Example workflows for drilling sequences are also schematized in Fig. 26 and Fig. 27, with the use of, respectively, extended shaft drill bits 1705, 1710 and 1720, and drilling guide extender 1850. The drill bits and the extenders are designed so that they can slide and fit into the socket until the drill bit/implant reaches its final position inside the patient's anatomy.

The drilling operation may be executed in conjunction with live tracking of the surgical instrument (for example drill 1780) used for the formation of the bone socket in order to check the congruency of the physical procedure with respect to pre-operative planning. The use of the guided drilling operation via a surgical template in conjunction with live tracking is a significant advantage.

The various components described can be made from a variety of materials or composites. In particular, but non-limiting embodiments, the drill bits 1705, 1710, 1720, 1805, 1810, 1820 and the guide-extender 1859 can be made of stainless steel. The collar 1750 can be made of a polymer material, such as polytetrafluoroethylene (PTFE) (sold under the brand name Teflon™).

### Software Application

Images of volume slices displayed in one or more window(s) to the user by the navigation software represent an important aspect of the computer-implemented surgical navigation method and/or system. Images of volume slices play an important role in two stages of the computer-implemented method: the virtual planning phase and the surgical navigation phase. A general description of preferred image views is provided below with reference to Figs. 28 to 33.

In one example there is provided a system for surgical navigation or planning. The system comprises at least one processing system for obtaining a three-dimensional anatomical representation of part of a patient, as well as for producing at least one two-dimensional volume slice view of the three-dimensional anatomical representation. The at least one two-dimensional volume slice view is based on or determined at least partially from a physical position or axial orientation of a surgical instrument, or a virtual position or axial orientation of a surgical implant. A display device is included for displaying the at least one two-dimensional volume slice view to a user, such as a surgeon. The at least one two-dimensional volume slice view, to be displayed to the user, can be updated, transformed or otherwise revised or refreshed by movement of the physical position or axial orientation of the surgical instrument during surgical navigation, or by movement of the virtual position or axial orientation of the surgical implant during surgical planning (noting virtual implants are generally fixed in position during subsequent surgical navigation). The display device is able to simultaneously display a view of the three-dimensional anatomical representation and the at least one two-dimensional volume slice view to the user, for example as shown in Figs. 28 and 29.

In another example, the at least one processing system is able to produce two or more two-dimensional volume slice views, and the display device is able to simultaneously display the two or more two-dimensional volume slice views to the user, again for example as shown in Figs. 28 and 29. The system can also include, for surgical navigation, a tracking device, such as one or more cameras, for recording a physical position and axial orientation of at least one reference tool provided with one or more reference markers. The at least one processing system is able to transform the physical position and axial orientation of the at least one reference tool into spatial coordinates relative to the three-dimensional anatomical representation of part of the patient. Preferably, the at least one reference tool is a first reference tool able to be mounted on the surgical instrument and including one or more reference markers, as previously discussed. A second reference tool is preferably provided that is able to be mounted on a connector for attaching to an implant and including one or more reference markers.

Preferably, the connector is able to hold fiducial markers, such as radiopaque markers, in a fiducial or radiopaque markers component which enables a determination of the physical position of the radiopaque markers with respect to the patient. The tracking device can be provided as one or more cameras, such as one or more stereoscopic cameras. In use, the display device displays a real-time tracking view of the position and axial orientation of a tip of the surgical instrument relative to the three-dimensional anatomical representation and the at least one two-dimensional volume slice view. The position and axial orientation of the tip is presented together with one or more pre-planned implant positions, such as in the example presented in Fig. 29. The position of the tip can be used to determine a position of a nearest implant, so that the displayed at least one two-dimensional volume slice view is updated to be centered and aligned according to the axes of the nearest implant.

In various examples, the display device is able to show a display window simultaneously presenting one or more of:
a main two-dimensional volume slice view that is perpendicular to a panoramic profile;
an axial two-dimensional volume slice view;
an antero-posterior two-dimensional volume slice view;
a view of the three-dimensional anatomical representation; and/or
a real-time tracking view of the position and axial orientation of a tip of a surgical instrument.

In another example embodiment, there is provided a computer-implemented method for surgical navigation or planning. The method comprising a step of obtaining, by at least one computer system, a three-dimensional anatomical representation of part of a patient, and a further step of producing, by the at least one computer system, at least one two-dimensional volume slice view of the three-dimensional anatomical representation, the at least one two-dimensional volume slice view based on a physical position or axial orientation of a surgical instrument, or a virtual position or axial orientation of a surgical implant. The method also involves displaying, by a display device, the at least one two-dimensional volume slice view to a user, and, updating, by the at least one computer system, the displayed at least one two-dimensional volume slice view based on movement of the physical position or axial orientation of the surgical instrument during surgical navigation, or by movement of the virtual position or axial orientation of the surgical implant during surgical planning.

Optionally, though preferably, before the three-dimensional anatomical representation of part of the patient is obtained, a radiopaque markers plate is mounted on the connector. The user touches the surgical instrument at or near positions of the radiopaque markers to enable the at least one computer system to register a position and axial orientation of the surgical instrument with respect to the patient's real-world anatomy.

In further examples, the at least one two-dimensional volume slice view can be:
a main slice view providing a volume slice perpendicular to a panoramic profile;
an axial view providing a volume slice perpendicular to the main slice view and coplanar with the panoramic profile;
an antero-posterior view providing a volume slice perpendicular to the main slice view and tangential to the panoramic profile; and/or
part of a panoramic profile providing a reconstructed perspective view of multiple layers of volume slice views using parallel panoramic profiles.

In still further examples, the at least one two-dimensional volume slice view can show:
a representation of a surgical instrument in a live-tracked position and a representation of one or more implants in virtually planned positions;
a section of an implant, abutment or tooth;
in surgical planning, a landmark for positioning a virtual implant;
a representation of a panoramic profile; and/or
a representation of a contour of other views.

'3D views' provide volume rendering via different techniques corresponding to different known algorithms. These 3D representations or images, for example images 1910 or 1965, can include:
patient's anatomy obtained from a CT scan;
implants as planned in an virtual implant planning phase;
virtual surgical instrument tip when in navigation mode;
implant abutments, virtual teeth, circles and arrows used to spatially transform the objects in 3D.

'Main slice views' provide a volume slice perpendicular to a panoramic profile. These 2D images for example images 1920 or 1975, can include:
a representation of a surgical instrument in its live-tracked position and can include a representation of implants in their virtually planned position;
a section of an implant/abutment/tooth if they are in the probed area of the volume;
in the implant planning phase, the user places landmark(s) for positioning the virtual implant, selecting from an implant/teeth/abutments library.

'Axial views' provide a volume slice perpendicular to the main slice view and coplanar with the panoramic profile plane. These 2D images for example images 1930 or 1980, can include:
a representation of the surgical instrument in its live-tracked position and can include a representation of implants in their virtually planned position;
a section of an implant/abutment/tooth if they are in the probed area of the volume;
a representation of the panoramic profile.

'Antero-posterior views' provide a volume slice perpendicular to the main slice view and tangential to the panoramic profile. These 2D images for example images 1940 or 1985, can include:
a representation of the surgical instrument in its live-tracked position and can include a representation of implants in their virtually planned position;
a section of an implant/abutment/tooth if they are in the probed area of the volume.

'Panoramic views' provide an artificially reconstructed perspective view of multiple layers of volume slices probed using parallel panoramic profiles. These 3D images for example image 1970 can include:
a representation of the contour of other views;
a representation of the surgical instrument in its live-tracked position and can include a representation of implants in their virtually planned position;
a section of an implant/abutment/tooth if they are in the probed area of the volume.

In the navigation phase the views can be updated in two ways. Referring to Fig. 28, a display window 1900 shows the intersection of the main slice view 1920, the axial view 1930 and the antero-posterior views 1940 is coincidental with the live-tracked 3D position of the surgical drill tip within the patient's anatomy (as shown in Fig. 28). Alternatively, the main view, the axial view and the antero-posterior views are aligned to the axes of a specific implant. This can be one of the implants positioned during the virtual planning phase. If more than one implant is present, as shown in Fig. 29 on display window 1950, an algorithm can be used to select the implant closer to the live-tracked 3D position of the surgical drill tip.

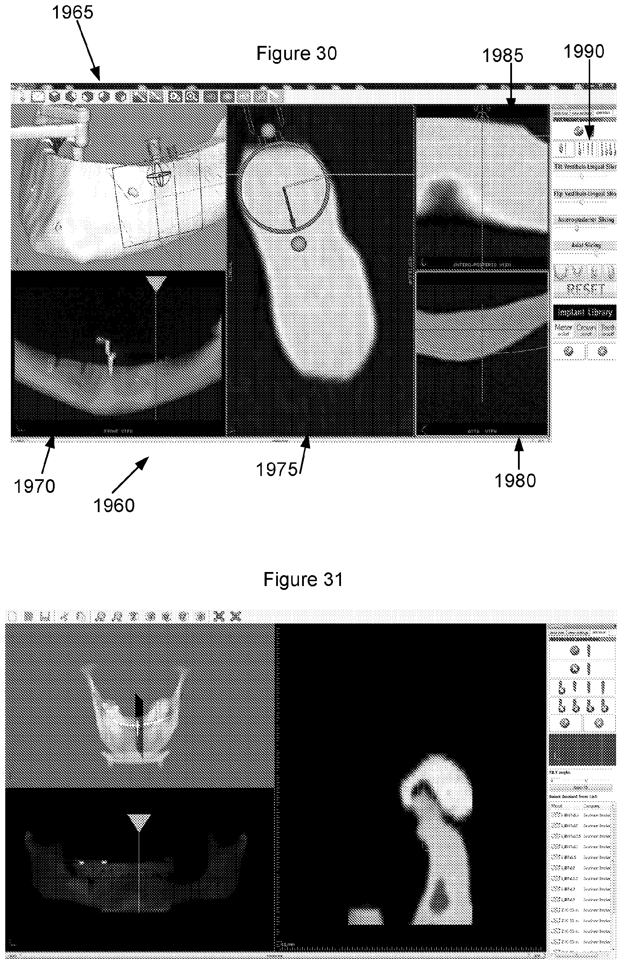

In the virtual planning phase, and example screen shot being illustrated in Fig. 30, the views in display window 1960 are updated by sliding bars present in the user interface 1990 which are linked to the following transformations:
Scroll perpendicular to the panoramic profile;
Scroll parallel to the antero-posterior slice;
Scroll parallel to the axial slice;
Tilt along the main slice axis;
Flip along the main slice axis; and/or
Rotation of the panoramic profile around its axis.

The software application includes functions for planning of surgery in a virtual environment. Following a guided procedure the user can perform the following actions:
Select an implant from an implants library;
Design a customised implant via geometry specifications and assign a name/company name to the customised implant;
Select a volume slice via transformation sliding bars present in the user interface 1990 and place the desired start and end point of the implant;
Move the implant in 3D via dedicated arrows and circles;
Pre-visualise an abutment on top of the implant placed;
Pre-visualise, manipulate and spatially transform a tooth on top of the implant placed via selection from a teeth library.

As part of the navigation system, the tracking device, e.g. a tracking camera, tracks the reference tools with a certain margin of error. As part of the system, a tracking algorithm based on known neural network algorithms can be applied in order to minimise or reduce this error. The tracking algorithm is applied to the positions read by the tracking device as a filter before the information is passed to a positional rendering engine of the software application.

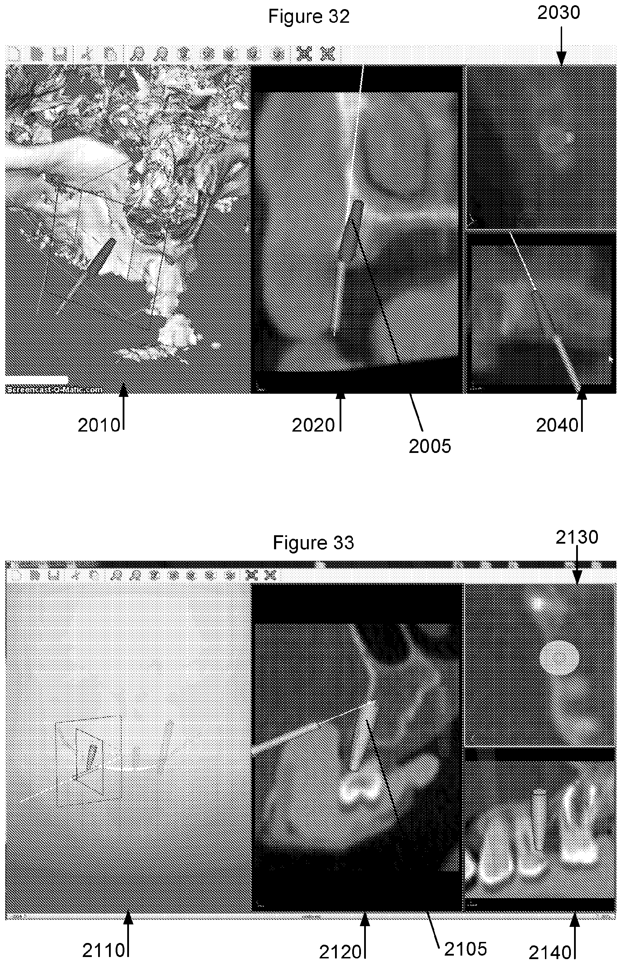

Once the registration procedure has been visually confirmed in a navigation panel of the software application, the user can visualize live or real-time tracking of the position of the tip and the axis or orientation of the surgical instrument onto the patient's tomographic scan. Fig. 31 is a sample screenshot from the software application in which the interface for the main virtual implant planning stage is displayed. Figs. 32 and 33 are sample screenshots from the software application taken during a main navigation operation during surgery. Referring to Fig. 32 showing implant 2005, information displayed to the user is, optionally, composed of one, two, three, four or more views, for example:
(i) a 3D view 2010 of a volume reconstruction of the patient's scan plus implants in their planned position;
(ii) an axial slice view 2030 of the volume;
(iii) a slice view 2020 which is perpendicular to the panoramic profile;
(iv) an additional slice view 2040 along a customized axis.

A curved section view, for example a panoramic view created sectioning the volume along the panoramic profile, which shows the current position of the surgical instrument and the planned implants can also be embedded. Similarly, referring to Fig. 33, showing implant 2105, information displayed to the user can include: (i) a 3D view 2110 with implants in their planned position; (ii) an axial slice view 2130 of the volume; (iii) a slice view 2120 which is perpendicular to the panoramic profile; (iv) an additional view 2140 along a customized axis or direction.

The function allowing the position of the volume slices represented in the views to be updated is a significant advantage during a surgical procedure. The updates in views are preferably triggered by movement of the surgical instrument within the scanned volume. The position of the surgical instrument is used by the software application to select the corresponding slice of the volume and visualize it in multiple views. Each one of the slice views represents scalars in gray values of the probed volume in that particular position. Each one of the slices also represents the current selected implant. The axes of the slices can be aligned with the axis of current selected implant in order to facilitate the alignment of the imaged surgical tool with the implant. The software determines that an implant is current calculating the closest distance between the position of the probe and the different implants.

The virtualized position of the tip of the surgical instrument is used to determine the updates in views presented to the user according to the nearest implant so that the displayed at least one two-dimensional volume slice view is updated, preferably automatically, to be centered and aligned according to the axes of the nearest implant location. Both the automation of the implant selection and the alignment of the slice views with the implant axes provide significant advantages in surgical use. Additional guidance is provided for the physical implant placement when the software application is used, and the accuracy of the procedure is maximized when the user aligns the surgical instrument with the virtual implant in all the slice views presented to the user.

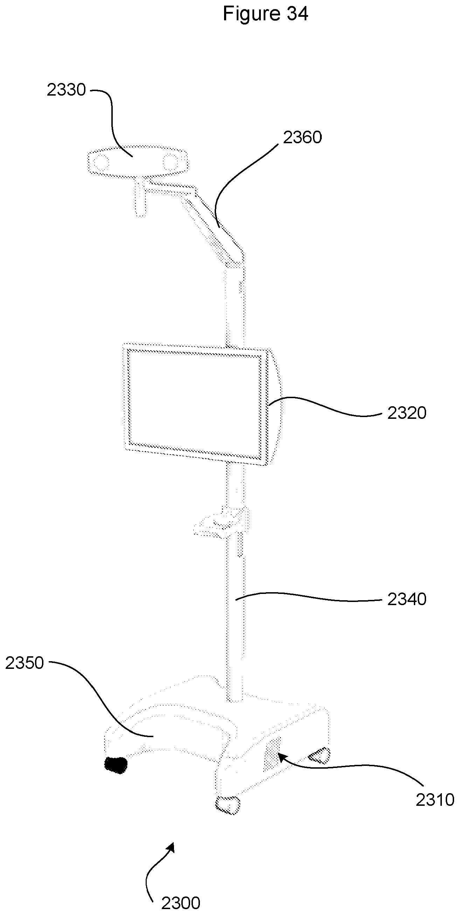

Fig. 34 illustrates an example system 2300 or integrated device for surgical planning and/or navigation. A processing unit 2310, for example computer 30 or processing system 50, is mounted in base 2350. Support 2340 is fixed to base 2350. Attached to support 2340 is monitor 2320, preferably a touch screen monitor that can also act as a user interface. Tracking camera(s) 2330 is attached to and supported by moveable arm 2360 which is attached to or forms part of support 2340. Optionally, system 2300 is moveable for ease of use, for example being provided on wheels.

Although a preferred embodiment has been described in detail, it should be understood that many modifications, changes, substitutions or alterations will be apparent to those skilled in the art without departing from the scope of the present invention.

The present invention in various embodiments may take the form of a computer-implemented method, a computing system or processor, hardware, software or a computer program product, firmware, or a combination of software and hardware aspects.

It should be appreciated that throughout the description, discussions utilizing terms such as "processing" or "computing" or "calculating" or "determining" or "displaying" or the like, refer to the action and processes of a computer, computer system, processor or similar electronic computing device, that manipulates and transforms data represented as physical (electronic) quantities within the computer system's processors, memories or registers or other such information storage, transmission or display devices.

## Claims

1. A system (10, 2300) for surgical navigation in oral surgery, comprising:
at least one processing system (30, 50, 2310) for:
obtaining a three-dimensional anatomical representation (1910, 1965, 1970, 2010, 2110) of part of a patient; and
producing at least one two-dimensional volume slice view (1920, 1930, 1940, 1975, 1980, 1985, 2020, 2030, 2040, 2120, 2130, 2140) of the three-dimensional anatomical representation (1910, 1965, 1970, 2010, 2110), the at least one two-dimensional volume slice view (1920, 1930, 1940, 1975, 1980, 1985, 2020, 2030, 2040, 2120, 2130, 2140) based on a physical position and axial orientation of at least one reference tool (26, 28, 800, 860, 870, 880, 1000);
a display device (14, 58, 2320) for displaying the at least one two-dimensional volume slice view (1920, 1930, 1940, 1975, 1980, 1985, 2020, 2030, 2040, 2120, 2130, 2140) to a user;
a tracking device (24, 2330) for recording the physical position and axial orientation of the at least one reference tool (26, 28, 800, 860, 870, 880, 1000) provided with one or more reference markers (805);
wherein the at least one processing system (30, 50, 2310) is able to transform the physical position and axial orientation of the at least one reference tool (26, 28, 800, 860, 870, 880, 1000) into spatial coordinates relative to the three-dimensional anatomical representation (1910, 1965, 1970, 2010, 2110) of part of the patient;
wherein, the at least one two-dimensional volume slice view (1920, 1930, 1940, 1975, 1980, 1985, 2020, 2030, 2040, 2120, 2130, 2140) to be displayed is updated by movement of the physical position and axial orientation of the at least one reference tool (26, 28, 800, 860, 870, 880, 1000) during surgical navigation in oral surgery; **characterised by**
the at least one reference tool (26, 28, 800, 860, 870, 880, 1000) being a first reference tool (26, 1000) mounted on a piezo-electric surgical instrument (1010, 1040, 1780, 1880); and
a drill tip (1005, 1050) mounted on the piezo-electric surgical instrument (1010, 1040, 1780, 1880);
wherein in use the display device (14, 58, 2320) displays a real-time tracking view of the position and axial orientation of the drill tip (1005, 1050) of the piezo-electric surgical instrument (1010, 1040, 1780, 1880) relative to the three-dimensional anatomical representation (1910, 1965, 1970, 2010, 2110) and the at least one two-dimensional volume slice view (1920, 1930, 1940, 1975, 1980, 1985, 2020, 2030, 2040, 2120, 2130, 2140).

2. The system of claim 1, wherein the display device (14, 58, 2320) is able to simultaneously display a view of the three-dimensional anatomical representation (1910, 1965, 1970, 2010, 2110) and the at least one two-dimensional volume slice view (1920, 1930, 1940, 1975, 1980, 1985, 2020, 2030, 2040, 2120, 2130, 2140) to the user.

3. The system of claim 1 or 2, wherein the at least one processing system (30, 50,2310) is able to produce two or more two-dimensional volume slice views (1920, 1930, 1940, 1975, 1980, 1985, 2020,2030, 2040,2120, 2130, 2140), and the display device (14, 58, 2320) is able to simultaneously display the two or more two-dimensional volume slice views (1920, 1930, 1940, 1975, 1980, 1985, 2020, 2030, 2040, 2120, 2130, 2140) to the user.

4. The system of claim 1, further including a second reference tool (28, 800, 860, 870, 880) able to be mounted on a connector (605, 705) for attaching to an implant (2005, 2105) and including one or more reference markers (805).

5. The system of claim 4, wherein a fiducial markers plate (1100), which holds fiducial markers, is mounted on the connector (605, 705) and enables a determination of the physical position of the fiducial markers with respect to the patient.

6. The system of claim 1, wherein the tracking device (24, 2330) includes one or more cameras or one or more stereoscopic cameras.

7. The system of claim 1, wherein the position and axial orientation of the drill tip (1005, 1050) is presented together with one or more pre-planned implant positions.

8. A computer-implemented method (100, 200, 300) for surgical planning of oral surgery, the method (100, 200, 300) comprising:
obtaining, by at least one computer system (30, 50, 2310), a three-dimensional anatomical representation (1910, 1965, 1970, 2010, 2110) of part of a patient; and **characterised by**,
producing, by the at least one computer system (30, 50, 2310), at least one two-dimensional volume slice view (1920, 1930, 1940, 1975, 1980, 1985, 2020, 2030, 2040, 2120, 2130, 2140) of the three-dimensional anatomical representation (1910, 1965, 1970, 2010, 2110), the at least one two-dimensional volume slice view (1920, 1930, 1940, 1975, 1980, 1985, 2020, 2030, 2040, 2120, 2130, 2140) based on a virtual position and axial orientation of a surgical implant (2005, 2105);
displaying, by a display device (14, 58, 2320), the at least one two-dimensional volume slice view (1920, 1930, 1940, 1975, 1980, 1985, 2020, 2030, 2040, 2120, 2130, 2140) to a user; and,
updating, by the at least one computer system (30, 50, 2310), the displayed at least one two-dimensional volume slice view (1920, 1930, 1940, 1975, 1980, 1985, 2020, 2030, 2040, 2120, 2130, 2140) based on movement of the virtual position and axial orientation of the surgical implant (2005, 2105) during surgical planning of oral surgery.

9. The computer-implemented method of claim 8, wherein the at least one two-dimensional volume slice view (1920, 1930, 1940, 1975, 1980, 1985, 2020, 2030, 2040,2120,2130,2140) is:
an axial slice view of the volume;
a slice view perpendicular to a panoramic profile; and/or
a slice view along a customized axis.

## Patentansprüche

1. Ein System (10, 2300) für die chirurgische Navigation bei der Oralchirurgie, das folgende Merkmale aufweist:
zumindest ein Verarbeitungssystem (30, 50, 2310) zum:
Erhalten einer dreidimensionalen anatomischen Darstellung (1910, 1965, 1970, 2010, 2110) eines Körperteils eines Patienten; und
Erzeugen zumindest einer zweidimensionalen Volumenschnittansicht (1920, 1930, 1940, 1975, 1980, 1985, 2020, 2030, 2040, 2120, 2130, 2140) der dreidimensionalen anatomischen Darstellung (1910, 1965, 1970, 2010, 2110), wobei die zumindest eine zweidimensionale Volumenschnittansicht (1920, 1930, 1940, 1975, 1980, 1985, 2020, 2030, 2040, 2120, 2130, 2140) auf einer physischen Position und Achsenausrichtung zumindest eines Referenzinstruments (26, 28, 800, 860, 870, 880, 1000) basiert;
eine Anzeigevorrichtung (14, 58, 2320) zum Anzeigen der zumindest einen zweidimensionalen Volumenschnittansicht (1920, 1930, 1940, 1975, 1980, 1985, 2020, 2030, 2040, 2120, 2130, 2140) für einen Benutzer;
eine Verfolgungsvorrichtung (24, 2330) zum Aufzeichnen der physischen Position und Achsenausrichtung des zumindest einen Referenzinstruments (26, 28, 800, 860, 870, 880, 1000), das mit einem oder mehreren Referenzmarkierern (805) versehen ist;
wobei das zumindest eine Verarbeitungssystem (30, 50, 2310) in der Lage ist, die physische Position und Achsenausrichtung des zumindest einen Referenzinstruments (26, 28, 800, 860, 870, 880, 1000) in räumliche Koordinaten relativ zu der dreidimensionalen anatomischen Darstellung (1910, 1965, 1970, 2010, 2110) eines Körperteils des Patienten umzuwandeln;
wobei die zumindest eine zweidimensionale Volumenschnittansicht (1920, 1930, 1940, 1975, 1980, 1985, 2020, 2030, 2040, 2120, 2130, 2140), die angezeigt werden soll, durch eine Verschiebung der physischen Position und Achsenausrichtung des zumindest einen Referenzinstruments (26, 28, 800, 860, 870, 880, 1000) während der chirurgischen Navigation bei der Oralchirurgie aktualisiert wird;
**gekennzeichnet durch**
das zumindest eine Referenzinstrument (26, 28, 800, 860, 870, 880, 1000), das ein erstes Referenzinstrument (26, 1000) ist, das auf einem piezoelektrischen chirurgischen Instrument (1010, 1040, 1780, 1880) befestigt ist; und
eine Bohrspitze (1005, 1050), die auf dem piezoelektrischen chirurgischen Instrument (1010, 1040, 1780, 1880) befestigt ist;
wobei die Anzeigevorrichtung (14, 58, 2320) bei Gebrauch eine Echtzeit-Verfolgungsansicht der Position und Achsenausrichtung der Bohrspitze (1005, 1050) des piezoelektrischen chirurgischen Instruments (1010, 1040, 1780, 1880) relativ zu der dreidimensionalen anatomischen Darstellung (1910, 1965, 1970, 2010, 2110) und der zumindest einen zweidimensionalen Volumenschnittansicht (1920, 1930, 1940, 1975, 1980, 1985, 2020, 2030, 2040, 2120, 2130, 2140) anzeigt.

2. Das System gemäß Anspruch 1, wobei die Anzeigevorrichtung (14, 58, 2320) in der Lage ist, dem Benutzer gleichzeitig eine Ansicht der dreidimensionalen anatomischen Darstellung (1910, 1965, 1970, 2010, 2110) und der zumindest einen zweidimensionalen Volumenschnittansicht (1920, 1930, 1940, 1975, 1980, 1985, 2020, 2030, 2040, 2120, 2130, 2140) anzuzeigen.

3. Das System gemäß Anspruch 1 oder 2, wobei das zumindest eine Verarbeitungssystem (30, 50, 2310) in der Lage ist, zwei oder mehr zweidimensionale Volumenschnittansichten (1920, 1930, 1940, 1975, 1980, 1985, 2020, 2030, 2040, 2120, 2130, 2140) zu erzeugen, und die Anzeigevorrichtung (14, 58, 2320) in der Lage ist, dem Benutzer gleichzeitig die zwei oder mehr zweidimensionalen Volumenschnittansichten (1920, 1930, 1940, 1975, 1980, 1985, 2020, 2030, 2040, 2120, 2130, 2140) anzuzeigen.

4. Das System gemäß Anspruch 1, das ferner ein zweites Referenzinstrument (28, 800, 860, 870, 880) umfasst, das zum Anbringen an einem Implantat (2005, 2105) auf einem Verbinder (605, 705) befestigt sein kann und einen oder mehrere Referenzmarkierer (805) umfasst.

5. Das System gemäß Anspruch 4, wobei eine Justiermarkiererplatte (1100), die Justiermarkierer hält, auf dem Verbinder (605, 705) befestigt ist und ein Bestimmen der physischen Position der Justiermarkierer in Bezug auf den Patienten ermöglichst.

6. Das System gemäß Anspruch 1, wobei die Verfolgungsvorrichtung (24, 2330) eine oder mehrere Kameras oder eine oder mehrere Stereokameras umfasst.

7. Das System gemäß Anspruch 1, wobei die Position und Achsenausrichtung der Bohrspitze (1005, 1050) zusammen mit einem oder mehreren vorab geplanten Implantatpositionen präsentiert werden.

8. Ein computerimplementiertes Verfahren (100, 200, 300) für die chirurgische Planung von Oralchirurgie, wobei das Verfahren (100, 200, 300) folgende Schritte aufweist:
Erhalten, durch zumindest ein Computersystem (30, 50, 2310), einer dreidimensionalen anatomischen Darstellung (1910, 1965, 1970, 2010, 2110) eines Körperteils eines Patienten; und
**gekennzeichnet durch**
Erzeugen, durch das zumindest eine Computersystem (30, 50, 2310), zumindest einer zweidimensionalen Volumenschnittansicht (1920, 1930, 1940, 1975, 1980, 1985, 2020, 2030, 2040, 2120, 2130, 2140) der dreidimensionalen anatomischen Darstellung (1910, 1965, 1970, 2010, 2110), wobei die zumindest eine zweidimensionale Volumenschnittansicht (1920, 1930, 1940, 1975, 1980, 1985, 2020, 2030, 2040, 2120, 2130, 2140) auf einer virtuellen Position und Achsenausrichtung eines chirurgischen Implantats (2005, 2105) basiert;
Anzeigen, durch eine Anzeigevorrichtung (14, 58, 2320), der zumindest einen zweidimensionalen Volumenschnittansicht (1920, 1930, 1940, 1975, 1980, 1985, 2020, 2030, 2040, 2120, 2130, 2140) für einen Benutzer; und
Aktualisieren, durch das zumindest eine Computersystem (30, 50, 2310), der angezeigten zumindest einen zweidimensionalen Volumenschnittansicht (1920, 1930, 1940, 1975, 1980, 1985, 2020, 2030, 2040, 2120, 2130, 2140) auf Basis einer Verschiebung der virtuellen Position und Achsenausrichtung des chirurgischen Implantats (2005, 2105) während der chirurgischen Planung einer Oralchirurgie.

9. Das computerimplementierte Verfahren gemäß Anspruch 8, wobei die zumindest eine zweidimensionale Volumenschnittansicht (1920, 1930, 1940, 1975, 1980, 1985, 2020, 2030, 2040, 2120, 2130, 2140) Folgendes ist:
eine axiale Schnittansicht des Volumens;
eine Schnittansicht, die senkrecht zu einem Panoramaprofil ist; und/oder
eine Schnittansicht entlang einer benutzerdefinierten Achse.

## Revendications

1. Système (10, 2300) pour la navigation chirurgicale en chirurgie buccale, comprenant:
au moins un système de traitement (30, 50, 2310) destiné à:
obtenir une représentation anatomique tridimensionnelle (1910, 1965, 1970, 2010, 2110) d'une partie d'un patient; et
produire au moins une vue en coupe de volume bidimensionnelle (1920, 1930, 1940, 1975, 1980, 1985, 2020, 2030, 2040, 2120, 2130, 2140) de la représentation anatomique tridimensionnelle (1910, 1965, 1970, 2010, 2110), l'au moins une vue en coupe de volume bidimensionnelle (1920, 1930, 1940, 1975, 1980, 1985, 2020, 2030, 2040, 2120, 2130, 2140) étant basée sur une position physique et une orientation axiale d'au moins un outil de référence (26, 28, 800, 860, 870, 880, 1000);
un dispositif d'affichage (14, 58, 2320) destiné à afficher l'au moins une vue en coupe de volume bidimensionnelle (1920, 1930, 1940, 1975, 1980, 1985, 2020, 2030, 2040, 2120, 2130, 2140) à un utilisateur;
un dispositif de suivi (24, 2330) destiné à enregistrer la position physique et l'orientation axiale de l'au moins un outil de référence (26, 28, 800, 860, 870, 880, 1000) pourvu d'un ou plusieurs repères de référence (805);
dans lequel l'au moins un système de traitement (30, 50, 2310) est apte à transformer la position physique et l'orientation axiale de l'au moins un outil de référence (26, 28, 800, 860, 870, 880, 1000) en coordonnées spatiales relatives à la représentation anatomique tridimensionnelle (1910, 1965, 1970, 2010, 2110) d'une partie du patient;
dans lequel l'au moins une vue en coupe de volume bidimensionnelle (1920, 1930, 1940, 1975, 1980, 1985, 2020, 2030, 2040, 2120, 2130, 2140) à afficher est mise à jour par le déplacement de la position physique et de l'orientation axiale de l'au moins un outil de référence (26, 28, 800, 860, 870, 880, 1000) pendant la navigation chirurgicale en chirurgie buccale;
**caractérisé par le fait que**
l'au moins un outil de référence (26, 28, 800, 860, 870, 880, 1000) est un premier outil de référence (26, 1000) monté sur un instrument chirurgical piézoélectrique (1010, 1040, 1780, 1880); et
une pointe de forage (1005, 1050) est montée sur l'instrument chirurgical piézoélectrique (1010, 1040, 1780, 1880);
dans lequel, en utilisation, le dispositif d'affichage (14, 58, 2320) affiche une vue de suivi en temps réel de la position et de l'orientation axiale de la pointe de forage (1005, 1050) de l'instrument chirurgical piézoélectrique (1010, 1040, 1780, 1880) par rapport à la représentation anatomique tridimensionnelle (1910, 1965, 1970, 2010, 2110) et l'au moins une vue en coupe de volume bidimensionnelle (1920, 1930, 1940, 1975, 1980, 1985, 2020, 2030, 2040, 2120, 2130, 2140).

2. Système selon la revendication 1, dans lequel le dispositif d'affichage (14, 58, 2320) est apte à afficher simultanément une vue de la représentation anatomique tridimensionnelle (1910, 1965, 1970, 2010, 2110) et l'au moins une vue en coupe de volume bidimensionnelle (1920, 1930, 1940, 1975, 1980, 1985, 2020, 2030, 2040, 2120, 2130, 2140) à l'utilisateur.

3. Système selon la revendication 1 ou 2, dans lequel l'au moins un système de traitement (30, 50, 2310) est apte à produire deux ou plusieurs vues en coupe de volume bidimensionnelles (1920, 1930, 1940, 1975, 1980, 1985, 2020, 2030, 2040, 2120, 2130, 2140) et le dispositif d'affichage (14, 58, 2320) est apte à afficher simultanément les deux ou plusieurs vues en coupe de volume bidimensionnelles (1920, 1930, 1940, 1975, 1980, 1985, 2020, 2030, 2040, 2120, 2130, 2140) à l'utilisateur.

4. Système selon la revendication 1, comportant par ailleurs un deuxième outil de référence (28, 800, 860, 870, 880) apte à être monté sur un connecteur (605, 705) pour être fixé à un implant (2005, 2105) et comportant un ou plusieurs repères de référence (805).

5. Système selon la revendication 4, dans lequel une plaque à repères de référence (1100), qui présente des repères, est montée sur le connecteur (605, 705) et permet une détermination de la position physique des repères de référence par rapport au patient.

6. Système selon la revendication 1, dans lequel le dispositif de suivi (24, 2330) comporte une ou plusieurs caméras ou une ou plusieurs caméras stéréoscopiques.

7. Système selon la revendication 1, dans lequel la position et l'orientation axiale de la pointe de forage (1005, 1050) sont présentées ensemble avec une ou plusieurs positions d'implant pré-planifiées.

8. Procédé mis en œuvre par ordinateur (100, 200, 300) pour la planification chirurgicale d'une chirurgie buccale, le procédé (100, 200, 300) comprenant le fait de:
obtenir, par au moins un système d'ordinateur (30, 50, 2310), une représentation anatomique tridimensionnelle (1910, 1965, 1970, 2010, 2110) d'une partie d'un patient;
et **caractérisé par** le fait de
produire, par au moins un système d'ordinateur (30, 50, 2310), au moins une vue en coupe de volume bidimensionnelle (1920, 1930, 1940, 1975, 1980, 1985, 2020, 2030, 2040, 2120, 2130, 2140) de la représentation anatomique tridimensionnelle (1910, 1965, 1970, 2010, 2110), l'au moins une vue en coupe de volume bidimensionnelle (1920, 1930, 1940, 1975, 1980, 1985, 2020, 2030, 2040, 2120, 2130, 2140) étant basée sur une position virtuelle et une orientation axiale d'un implant chirurgical (2005, 2105);
afficher, par un dispositif d'affichage (14, 58, 2320), l'au moins une vue en coupe de volume bidimensionnelle (1920, 1930, 1940, 1975, 1980, 1985, 2020, 2030, 2040, 2120, 2130, 2140) à un utilisateur; et
mettre à jour, par au moins un système d'ordinateur (30, 50, 2310), l'affichage d'au moins une vue en coupe de volume bidimensionnelle (1920, 1930, 1940, 1975, 1980, 1985, 2020, 2030, 2040, 2120, 2130, 2140) sur base du déplacement de la position virtuelle et de l'orientation axiale de l'implant chirurgical (2005, 2105) pendant la planification chirurgicale de la chirurgie buccale.

9. Procédé mis en œuvre par ordinateur selon la revendication 8, dans lequel l'au moins une vue en coupe de volume bidimensionnelle (1920, 1930, 1940, 1975, 1980, 1985, 2020, 2030, 2040, 2120, 2130, 2140) est:
une vue en coupe axiale du volume;
une vue en coupe perpendiculaire à un profil panoramique; et/ou
une vue en coupe le long d'un axe personnalisé.
